# EUROPÄISCHE PATENTSCHRIFT

(11) **EP 2 568 953 B1**
(45) Veröffentlichungstag und Bekanntmachung des Hinweises auf die Patenterteilung: **14.05.2014**
(21) Anmeldenummer: 11714703.3
(22) Anmeldetag: 13.04.2011
(51) Int. Cl.: A61K 8/35, A61K 8/49, A61Q 17/04, C11B 5/00

(54) **TRIAZINE ALS REAKTIONSBESCHLEUNIGER**
TRIAZINES AS REACTION ACCELERATORS
TRIAZINES UTILISÉES COMME ACCÉLÉRATEURS DE RÉACTION

(30) Priorität: 12.05.2010 EP 10005033
(43) Veröffentlichungstag der Anmeldung: 20.03.2013
(73) Patentinhaber: Merck Patent GmbH, 64293 Darmstadt (DE)
(72) Erfinder: SCHEURICH, René, Peter, 64846 Gross-Zimmern (DE); RUDOLPH, Thomas, 64291 Darmstadt (DE)
(86) Internationale Anmeldenummer: PCT/EP2011/001872
(87) Internationale Veröffentlichungsnummer: WO 2011/141111

(56) Entgegenhaltungen:
- US-A1- 2009 246 158

## Beschreibung

Gegenstand der Erfindung ist die Verwendung von zumindest einer Verbindung nach Formel I und/oder deren Salz als Beschleuniger für eine photoinduzierte Reaktion von 3-(4-tert-Butyl-phenyl)-3-hydroxy-1-(4-methoxy-phenyl)-propan-1-on, 3-(4-Methoxyphenyl)-3-hydroxy-1-(4-tert-butyl-phenyl)-propan-1-on (oder synonym 1-(4-tert-Butyl-phenyl)-3-hydroxy-3-(4-methoxy-phenyl)-propan-1-on) oder Mischungen dieser beiden Verbindungen zu 1-(4-tert-Butylphenyl)-3-(4-methoxyphenyl)propan-1,3-dion. Die Verbindung 1-(4-tert-Butylphenyl)-3-(4-methoxyphenyl)propan-1,3-dion wird im Folgenden auch als Avobenzon bezeichnet. Des Weiteren betrifft die Erfindung eine Zubereitung, enthaltend zumindest eine Verbindung nach Formel I und ein Verfahren zur Stabilisierung der UV-Absorptionsfähigkeit einer Zubereitung mittels einer Mischung aus zumindest einer Verbindung nach Formel I zusammen mit 3-(4-Tert-butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen.

Die nachfolgenden Ausführungen zu einer der Substanzen 3-(4-Tert-butylphenyl)-3-hydroxy-1-(4-methoxy-phenyl)-propan-1-on oder 1-(4-tert-Butyl-phenyl)-3-hydroxy-3-(4-methoxy-phenyl)-propan-1-on gelten sinngemäß stets auch für die nicht genannte zweite Substanz oder für Mischungen der beiden Substanzen.

Strahlung der Sonne oder auch Strahlung von künstlichen Quellen, insbesondere Strahlung der Wellenlängen von 280 bis 400 Nanometer, sogenannte UV-Strahlung, kann mit Inhaltsstoffen von Zubereitungen und mit Materialien interagieren. Solche Inhaltsstoffe bzw. Materialien können Verbindungen wie zum Beispiel Pigmente, UV-Filter, Antioxidantien oder Kunststoffe sowie Polymere sein. Aufgrund der Absorption von Strahlung können jedoch solche Inhaltsstoffe bzw. Materialien durch zum Beispiel eine photochemische Reaktion eine strukturelle Veränderung erfahren. Eine solche photochemische Degradation von Inhaltsstoffen bzw. Materialien führt üblicherweise zu einer nicht erwünschten Verschlechterung der Zubereitung bzw. des Materials. Im Falle von Farbstoffen zum Beispiel, kann eine solche photochemische Degradation zu einem Ausbleichen oder einem Glanzverlust führen und bei Verpackungsmaterialien kann der enthaltene Kunststoff spröde werden und somit seine schützende Funktion verlieren.

Des Weiteren unterliegt die menschliche Haut gewissen Alterungsprozessen, die teilweise auf exogene Faktoren zurückzuführen sind. Zu den exogenen Faktoren zählen insbesondere das Sonnenlicht oder künstliche Strahlungsquellen mit vergleichbarem Spektrum, sowie Verbindungen, die durch die Strahlung entstehen können, wie undefinierte reaktive Photoprodukte, die auch einen radikalischen oder ionischen Charakter aufweisen können.

Des Weiteren sind sowohl die Haut als auch Inhaltsstoffe, sowie Materialien einem gewissen oxidativen Stress durch die Umgebung ausgesetzt. Dieser oxidative Stress führt zu einer oxidativen Degradation, die wiederum ebenfalls zu einer unerwünschten Verschlechterung der Materialien bzw. Zubereitungen führen kann und die den Alterungsprozess der Haut verstärken kann.

Nun sind eine Vielzahl von organischen und anorganischen UV-Filtern und Antioxidantien bekannt, die UV-Strahlung absorbieren und freie Radikale abfangen können. Dadurch sind diese UV-Filter und Antioxidantien in der Lage, die menschliche Haut zu schützen.

Aufgrund der photochemischen Zersetzung der UV-Filter selbst durch z.B. die Strahlung der Sonne oder durch künstliche Strahlungsquellen mit vergleichbarem Spektrum, sinkt jedoch die Konzentrationen der UV-Filter während der Anwendung bzw. Verwendung kontinuierlich ab und somit der Schutz gegen die aggressive UV-Strahlung ebenfalls. Üblicherweise kann diesem Effekt durch besonders stabile UV-Filter entgegengewirkt werden, jedoch können auch diese UV-Filter einer gewissen photochemischen Degradation unterliegen. Zudem ist die Stabilität des jeweiligen UV-Filters auch abhängig von der jeweiligen Zubereitung, sodass nicht in jeder Formulierung eine ausreichende Stabilität des jeweiligen UV-Filters gegeben ist.

US 2009/246158 A1 (RUDOLPH THOMAS [DE] ET AL) 1. Oktober 2009 (2009-10-01) offenbart (Anspruch 10; Beispiele 3-4) die Antioxidantien 3-(4-tert-Butyl-phenyl)-3-hydroxy-1-(4-methoxy-phenyl)- propan-1 -on und 1 -(4-tert-Butyl-phenyl)-3-hydroxy-3-(4-methoxy-phenyl)- propan-1-on.

Dieses Dokument offenbart ausserdem ([0081, 87]) verschiedene Triazine.

Die vorliegende Erfindung beschäftigt sich nun mit dem Problem, den Schutz, insbesondere in/oder durch Zubereitungen, gegen Sonnen- oder sonnenähnliches Licht und/oder gegen eine oxidative Schädigung zu verbessern.

Erfindungsgemäß wird dieses Problem durch die Gegenstände der unabhängigen Ansprüche gelöst. Vorteilhafte Ausführungsformen sind Gegenstand der abhängigen Ansprüche.

Überraschenderweise wurde nun festgestellt, dass Verbindungen nach Formel I, somit einfach- oder mehrfachsubstituierte Triazine oder Melamine, als Beschleuniger für eine photoinduzierte Reaktion von 3-(4-Tert-butyl-phenyl)-3-hydroxy-1-(4-methoxy-phenyl)-propan-1-on oder 1-(4-tert-Butyl-phenyl)-3-hydroxy-3-(4-methoxy-phenyl)-propan-1-on wirken können.

Dabei versteht man unter der photoinduzierten Reaktion die Umwandlung beispielsweise von 3-(4-Tert-butyl-phenyl)-3-hydroxy-1-(4-methoxyphenyl)-propan-1-on zu Avobenzon bei Bestrahlung mit Sonnen- oder sonnenähnlichem Licht, wobei gemäß nachfolgendem Reaktionsschema A 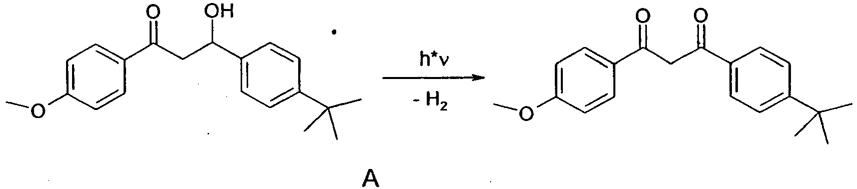 Wasserstoff abgegeben wird, der üblicherweise "in situ" durch umgebende Verbindungen abgefangen wird.

Somit kann durch Verwendung von Verbindungen nach Formel I die Bildung des UV-Filters Avobenzon durch photoinduzierte Reaktion aus 3-(4-Tert-butyl-phenyl)-3-hydroxy-1-(4-methoxy-phenyl)-propan-1-on oder 1-(4-tert-Butyl-phenyl)-3-hydroxy-3-(4-methoxy-phenyl)-propan-1-on oder Mischungen dieser beiden Verbindungen beschleunigt werden und deshalb aufgrund der beschleunigten Bildung von Avobenzon einer abnehmenden UV-Absorbtionsfähigkeit aufgrund eines Zerfalls von UV-Filtern z.B. in Zubereitungen zumindest teilweise entgegengewirkt werden.

Unter einem Beschleuniger versteht man somit eine Verbindung, insbesondere nach Formel I, wie nachfolgend beschrieben, die eine Reaktion von einem Ausgangsstoff zu einem Produkt beschleunigt, so dass das Produkt bei Vorhandensein eines Beschleunigers schneller gebildet wird, als bei Abwesenheit desselben.

Des Weiteren kann 3-(4-Tert-butyl-phenyl)-3-hydroxy-1-(4-methoxyphenyl)-propan-1-on aufgrund der photoinduzierten Reaktion zu Avobenzon rein formal gesehen Wasserstoff abgeben und damit eine gewisse antioxidative Wirkung aufzeigen, so dass 3-(4-Tert-butyl-phenyl)-3-hydroxy-1-(4-methoxy-phenyl)-propan-1-on zusammen mit einer Verbindung nach Formel I auch als Antioxidanz, insbesondere als Photoantioxidanz eingesetzt werden kann.

Unter einem Photoantioxidanz ist somit eine Verbindung zu verstehen, die bei Bestrahlung, insbesondere mit Licht einer Wellenlänge von 280 bis 400 Nanometern, eine antioxidante Wirkung aufzeigt.

Fortfolgend steht jeweils unabhängig voneinander:
Alk¹ für eine geradkettige oder verzweigte C₁- bis C₂₀-Alkylgruppe oder für eine geradkettige oder verzweigte C₂- bis C₂₀-Alkenylgruppe, die zumindest eine Doppelbindung aufweist oder für eine geradkettige oder verzweigte C₂- bis C₂₀-Alkinylgruppe, die zumindest eine Dreifachbindung aufweist, und/oder bei der zumindest ein oder mehrere nicht benachbarte C-Atome der Alkyl-, Alkenyl- oder Alkinylgruppe durch O oder Trimethylsilyl ersetzt sein kann/können und/oder die zumindest ein an ein primäres oder sekundäres C-Atom angebundenes OH aufweisen können;
Alk² für eine geradkettige oder verzweigte C₁- bis C₁₂-Alkylgruppe, die durch zumindest ein O unterbrochen sein kann,
Alk³ für eine geradkettige oder verzweigte C₁- bis C₁₂-Alkylgruppe, bei der zumindest ein oder mehrere nicht benachbarte C-Atome durch O ersetzt sein kann/können und/oder die zumindest ein an ein primäres oder sekundäres C-Atom angebundenes OH aufweisen kann;
Alk⁴ für eine geradkettige oder verzweigte C₁- bis C₈-Alkylgruppe;
Cyc¹ für eine C₃- bis C₈-Cycloalkyl-Gruppe, die zumindest eine Doppelbindung aufweisen kann, und/oder in der zumindest ein CH₂ durch O oder NH ersetzt sein kann;
Cyc² für eine C₅- bis C₆-Cycloalkyl-Gruppe, die zumindest eine Doppelbindung aufweisen kann, und/oder in der zumindest ein CH₂ durch O oder NH ersetzt sein kann;
Arl¹ für eine unsubstituierte, einfach- oder mehrfachsubstituierte C₆- bis C₂₀-Arylgruppe;
Arl² für eine unsubstituierte, einfach- oder mehrfachsubstituierte C₆- bis C₁₅-Arylgruppe;
Arl³ für eine unsubstituierte, einfach- oder mehrfachsubstituierte C₆- bis C₁₀-Arylgruppe;
Het¹ für eine unsubstituierte, einfach- oder mehrfachsubstituierte C₅- bis C₂₀-Arylgruppe, bei der zumindest ein CH₂ durch O, S oder NH ersetzt ist und steht damit für eine unsubstituierte, einfach- oder mehrfachsubstituierte C₅- bis C₂₀-Heteroarylgruppe,
Het² für eine unsubstituierte, einfach- oder mehrfachsubstituierte C₅- bis C₁₀-Arylgruppe, bei der zumindest ein CH₂ durch O, S oder NH ersetzt ist und steht damit für eine unsubstituierte, einfach- oder mehrfachsubstituierte C₅- bis C₁₀-Heteroarylgruppe;
Het³ für eine unsubstituierte, einfach- oder mehrfachsubstituierte C₅- bis C₆-Arylgruppe, bei der zumindest ein CH₂ durch O, S oder NH ersetzt ist und steht damit für eine unsubstituierte, einfach- oder mehrfachsubstituierte C₅- bis C₆-Heteroarylgruppe;
Eth¹ für -(CH₂-CH₂-O)ₘ₁-Alk² mit 1 ≤ m1 ≤ 16; wobei Alk² eine zuvor beschriebene Bedeutung hat,
Eth² für -(CH₂-CH₂-O)ₘ₁-Alk⁴ mit 1 ≤ m1 ≤ 8; wobei Alk⁴ eine zuvor beschriebene Bedeutung hat,
Eth³ für -(CH₂-CH₂-O)ₘ₁-Alk⁶ mit 1 ≤ m1 ≤ 4; wobei Alk⁶ eine zuvor beschriebene Bedeutung hat,
Hal für F, Cl, Br oder I.

Dabei umfasst eine:
geradkettige oder verzweigte C₁-C₄-Alkylgruppe Methyl, Ethyl, Propyl-, isoPropyl, Butyl, x-Methylpropyl (x=1;2) und tert.-Butyl;
geradkettige oder verzweigte C₁-C₈-Alkylgruppe die Substituenten der C₁-C₄-Alkylgruppe und Pentyl, Hexyl, Heptyl, Oktyl, x-Methylbutyl (x=1;2;3), x-Methylpentyl (x=1;2;3;4), x-Methylhexyl (x=1;2;3;4;5), x-Ethylpentyl (x=1;2;3), x-Ethylhexyl (x=1;2;3;4);
geradkettige oder verzweigte C₁-C₁₂-Alkylgruppe die Substituenten der C₁-C₈-Alkylgruppe und Nonyl, Decyl, Undecyl, Dodecyl;
geradkettige oder verzweigte C₁-C₂₀-Alkylgruppe die Substituenten der C₁-C₁₂-Alkylgruppe und Tridecyl, Tetradecyl, Pentadecyl, Hexadecyl, Heptadecyl, Oktadecyl, Nonadecyl, Eicosyl;
geradkettige oder verzweigte C₂-C₄-Alkenylgruppe Ethenyl, x-Propenyl (x=1;2), x-Butenyl (x=1;2;3) und Butadienyl;
geradkettige oder verzweigte C₂-C₈-Alkenylgruppe die Substituenten der C₂-C₄-Alkenylgruppe und x-Pentenyl (x=1;2;3;4), x-Hexenyl (x=1;2;3;4;5), x-Heptenyl (x=1;2;3;4;5;6), x-Oktenyl (x=1;2;3;4;5;6;7);
geradkettige oder verzweigte C₂-C₁₂-Alkenylgruppe die Substituenten der C₂-C₈-Alkenylgruppe und x-Nonenyl (x=1;2;3;4;5;6;7;8), x-Decenyl (x=1;2;3;4;5;6;7;8;9), x-Undecenyl (x=1;2;3;4;5;6;7;8;9;10), x-Dodecenyl (x=1;2;3;4;5;6;7;8;9;10;11);
geradkettige oder verzweigte C₂-C₂₀-Alkenylgruppe die Substituenten der C₂-C₁₂-Alkenylgruppe und 9-Oktadecenyl, 9,12-Oktadecadienyl, 9,12,15-Oktadecatrienyl, 10-Nonadecenyl, 10,13-Nonadecadienyl, 10,13,16-Nonadecatrienyl, 11-Eicosenyl, 11,14-Eicosadienyl, 11,14,17-Eicosatrienyl;
geradkettige oder verzweigte C₂-C₄-Alkinylgruppe Acetylenyl, x-Propinyl (x=1;2), x-Butinyl (x=1;2;3) und Butadiinyl;
geradkettige oder verzweigte C₂-C₈-Alkinylgruppe die Substituenten der C₂-C₄-Alkinylgruppe und x-Pentinyl (x=1;2;3;4), x-Hexinyl (x=1;2;3;4;5), x-Heptinyl (x=1;2;3;4;5;6), x-Oktinyl (x=1;2;3;4;5;6;7);
geradkettige oder verzweigte C₂-C₁₂-Alkinylgruppe die Substituenten der C₂-C₈-Alkinylgruppe und x-Noninyl (x=1;2;3;4;5;6;7;8), x-Decinyl (x=1;2;3;4;5;6;7;8;9), x-Undecinyl (x=1;2;3;4;5;6;7;8;9;10), x-Dodecinyl (x=1;2;3;4;5;6;7;8;9;10;11);
geradkettige oder verzweigte C₂-C₂₀-Alkinylgruppe die Substituenten der C₂-C₁₂-Alkinylgruppe und x-Oktadecinyl (x=1;17), x-Nonadecinyl (x=1;18), x-Eicosinyl (x=1;19);
C₅-C₆-Cycloalkylgruppe Cyclopentyl, x-Cyclopentenyl (x=1;2;3), x,y-Cyclopentadienyl (x,y=1,3;1,4;2,4), Cyclohexyl, x-Cyclohexenyl (x=1;2;3), x,y-Cyclohexadienyl (x,y=1,3;1,4;2,4;2,5), x-Tetrahydrofuranyl (x=2;3), x-(2,5-Dihydrofuranyl) (x=2;3), x-Tetrahydropyrrolyl (x=2;3), x-(2,5-Dihydropyrrolyl) (x=2;3), x-(4,5-Dihydrooxazolyl) (x=2;4;5), x-Oxazolidinyl (x=2;3;4;5), x-Piperidinyl (x=2;3;4;5), x-Pyranyl (x=2;3;4;5), x-(4,5-Dihydropyranyl) (x=2;3;4;5), x-Piperazinyl (x=2;3), x-(1,3-Dioxanyl) (x=2;4;5;6) und x-(1,4-Dioxanyl) (x=2;3;5;6);
C₃-C₈-Cycloalkylgruppe die Substituenten der C₅-C₆-Cycloalkylgruppe und Cyclopropyl, x-Cyclopropenyl (x=1;2), Cyclobutanyl, x-Cyclobutenyl (x=1;2;3), Cycloheptyl, Cyclooktyl, x-Methylcylclohexyl (x=2;3;4), x-Ethylcyclohexyl (x=2;3;4) und x,y-Dimethylcyclohexyl (x,y=2,3; 2,4);
unsubstituierte, einfach- oder mehrfach substituierte C₆-C₁₀-Arylgruppe, Phenyl, Naphthyl, x-Methylphenyl (x=o,m,p), x-Ethylphenyl (x=o,m,p), x-Propylphenyl (x=o,m,p), x-Isopropylphenyl (x=o,m,p), x-tert.-Butylphenyl (x=o,m,p), x-Nitrophenyl (x=o,m,p), x-Methoxyphenyl (x=o,m,p), x-Ethoxyphenyl (x=o,m,p), x-(Trifluormethyl)phenyl (x=o,m,p), x-(Trifluormethoxy)phenyl (x=o,m,p), x-(Trifluormethylsulfonyl)phenyl (x=o,m,p), x-Fluorphenyl (x=o,m,p), x-Chlorphenyl (x=o,m,p), x-Bromphenyl (x=o,m,p), x-Iodphenyl (x=o,m,p), x,y-Dimethylphenyl (x=2,3;2,4;2,5;2,6;3,4;3,5), x,y-Dihydroxyphenyl (x=2,3;2,4;2,5;2,6;3,4;3,5), x,y-Difluorphenyl (x=2,3;2,4;2,5;2,6;3,4;3,5), x,y-Dichlorphenyl (x=2,3;2,4;2,5;2,6;3,4;3,5), x,y-Dibromphenyl (x=2,3;2,4;2,5;2,6;3,4;3,5), x,y-Dimethoxyphenyl (x=2,3;2,4;2,5;2,6;3,4;3,5), 5-Fluor-2-methylphenyl, 3,4,5-Trimethoxyphenyl und 2,4,5-Trimethylphenyl;
unsubstituierte, einfach- oder mehrfach substituierte C₆-C₁₅-Arylgruppe, die C₆-C₁₀-Arylgruppe und Anthryl, Phenanthryl, Phenalenyl; unsubstituierte, einfach- oder mehrfach substituierte C₆-C₂₀-Arylgruppe, die C₆-C₁₅-Arylgruppe und Pyrenyl, Chrysenyl Naphthacenyl, Picenyl; wobei des Weiteren als Substituenten an der jeweilgen Arylgruppe Hal, Alk¹, Arl¹ Het¹ OH, OAlk¹, OArl¹, O Het¹ NH₂, NHAlk¹ NAlk¹₂, NHArl¹, NArl¹₂, NHHet¹, NHet¹₂, CN, COOH, COHal, CONHAlk¹, CONAlk¹₂, COOAlk¹, CONHArl¹, CONArl¹₂, COOArl¹, CONHHet¹, CONHet¹₂, COOHet¹, SO₂OH, SO₂Hal, SO₂CF₃, SO₂OAlk¹, SO₂NHAlk¹, SO₂NAlk¹₂, SO₂OArl¹, SO₂NHArl¹, SO₂NArl¹₂, SO₂OHet¹, SO₂NHHet¹, SO₂NHet¹₂, SCF₃, und NO₂ umfasst sind;
unsubstituierte, einfach- oder mehrfach substituierte C₅-C₆-Heteroarylgruppe, x-Furyl (x=2,3), x-Thienyl (x=2,3), x-Pyrrolyl (x=1,2,3), x-Imidazolyl (x=1,2,4,5), x-Pyrazolyl (x=3,4,5), x-Oxazolyl (x=2,4,5), x-Isoxazolyl (x=3,4,5), x-Thiazolyl (x=2,4,5), x-Isothiazolyl (x=3,4,5), x-Pyridyl (x=2,3,4), x-Pyrimidinyl (x=2,4,5,6), x-Pyrazinyl (x=2,3,5,6), x-Pyridazinyl (x=3,4,5,6), 1,2,3-Triazol-x-yl (x=1,4,5), 1,2,4-Triazol-x-yl (x=1,3,5), x-Tetrazolyl (x=1,5), 1,2,3-Oxadiazol-x-yl (x=4,5), 1,2,4-Oxadiazol-x-yl (x=3,5), 1,3,4-Thiadiazol-x-yl (x=2,5), 1,2,4-Thiadiazol-x-yl (x=3,5), 1,2,3-Thiadiazol-x-yl (x=4,5), x-2H-Thiopyranyl (x=2,3,4,5,6), x-4H-Thiopyranyl (x=2,3,4) und x-Pyrrolidinyl (x=1,2,3);
unsubstituierte, einfach- oder mehrfach substituierte C₆-C₁₀-Heteroarylgruppe, die C₆-C₁₀-Heteroarylgruppe und x-Benzofuryl (x=2,3,4,5,6,7), x-Benzothienyl (x=2,3,4,5,6,7), x-1H-Indolyl (x=1,2,3,4,5,6,7), x-Benzimidazolyl (x=1,2,4,5), x-Benzopyrazolyl (x=1,3,4,5,6,7), x-Benzoxazolyl (x=2,4,5,6,7), x-Benzisoxazolyl (x=3,4,5,6,7), x-Benzthiazolyl (x=2,4,5,6,7), x-Benzisothiazolyl (x=2,4,5,6,7), x-Benz-1,2,3-oxadiazolyl (x=4,5,6,7), x-Chinolinyl (x=1,2,3,4,5,6,7,8), x-Isochinolinyl (x=1,3,4,5,6,7,8), x-Chinazolinyl (x=2,4,5,6,7,8) und x-Benzo-1,2,3-triazolxl (x=1,6,7,8,9);
unsubstituierte, einfach- oder mehrfach substituierte C₆-C₂₀-Heteroarylgruppe, die C₆-C₁₀-Heteroarylgruppe und
x-Carbazolyl (x=1,2,3,4,9), x-Acridinyl (x=1,2,3,4,5,6,7,8,9) und x-Cinnolinyl (x=3,4,5,6,7,8);
wobei als Substiuenten an der jeweiligen Heteroarylgruppe Hal, Alk¹, Arl¹, Het¹ OH, OAlk¹, OArl¹, O Het¹, NH₂, NHAlk¹ NAlk¹₂, NHArl¹, NArl¹₂, NHHet¹, NHet¹₂, CN, COOH, COHal, CONHAlk¹, CONAlk¹₂, COOAlk¹, CONHArl¹, CONArl¹₂, COOArl¹, CONHHet¹ CONHet¹₂, COOHet¹, SO₂OH, SO₂Hal, SO₂CF₃, SO₂OAlk¹, SO₂NHAlk¹, SO₂NAlk¹₂, SO₂OArl¹, SO₂NHArl¹, SO₂NArl¹₂, SO₂OHet¹, SO₂NHHet¹ SO₂NHet¹₂, SCF₃, und NO₂ umfasst sind
Alk³ umfasst daher neben den zuvor gelisteten geradkettigen oder verzweigten Alkylgruppen mit 1 bis 12 C-Atomen auch insbesondere Alkylgruppen der Formel -CH₂-CH(OH)-CH₂-O-Alk⁴, wobei Alk⁴ eine der zuvor angegebenen Bedeutungen haben kann. Alk⁴ in der angegebenen Formel -CH₂-CH(OH)-CH₂-O-Alk⁴ ist bevorzugt Isopropyl.

Ein Gegenstand der Erfindung ist somit die Verwendung einer Verbindung nach Formel I 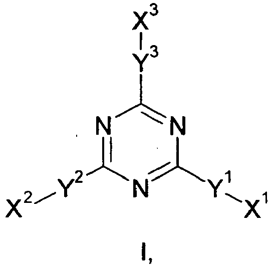
wobei Y¹ Y² und Y³ jeweils unabhängig voneinander stehen für eine Einfachbindung oder NH,
wobei X¹, X² und X³ jeweils unabhängig voneinander stehen für Alk¹ oder einen Substituenten der Formel II oder III 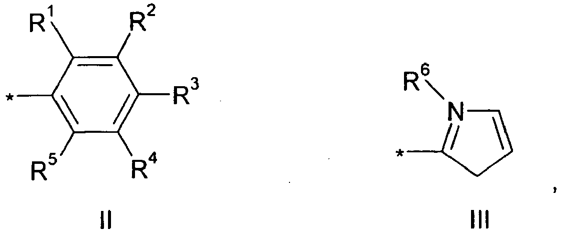
wobei R¹ bis R⁵ jeweils unabhängig voneinander stehen
für H, OH, Hal, Alk¹ OAlk¹, SAlk¹, NHAlk¹, N(Alk¹)₂, COOAlk¹, COOH, C(O)H, CONHAlk¹, CONH₂, COO⁻Kt⁺, Cyc¹, OCyc¹ Arl¹, OArl¹ COOArl¹, Biphenylyl, Het¹ OHet¹, Si(Alk²)₃, OEth, COOEth oder
für einen Substituenten der Formel IV 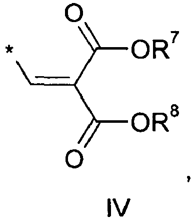
wobei Alk¹ jeweils unabhängig voneinander steht
für eine geradkettige oder verzweigte C₁- bis C₂₀-Alkylgruppe oder für eine geradkettige oder verzweigte C₂- bis C₂₀-Alkenylgruppe, die zumindest eine Doppelbindung aufweist oder für eine geradkettige oder verzweigte C₂- bis C₂₀-Alkinylgruppe, die zumindest eine Dreifachbindung aufweist, und/oder bei der zumindest ein oder mehrere nicht benachbarte C-Atome der Alkyl-, Alkenyl- oder Alkinylgruppe durch O oder Trimethylsilyl ersetzt sein kann/können und/oder die zumindest ein an ein primäres oder sekundäres C-Atom angebundenes OH aufweisen können,
wobei Cyc¹ jeweils unabhängig voneinander steht für eine C₃- bis C₈-Cycloalkyl-Gruppe, die zumindest eine Doppelbindung aufweisen kann, und/oder in der zumindest ein CH₂ durch O oder NH ersetzt sein kann, wobei Arl¹ jeweils unabhängig voneinander steht
für eine unsubstituierte, einfach- oder mehrfachsubstituierte C₆- bis C₂₀-Arylgruppe,
wobei Het¹ jeweils unabhängig voneinander steht für eine unsubstituierte, einfach- oder mehrfachsubstituierte C₅- bis C₂₀-Arylgruppe, bei der zumindest ein CH₂ durch O, S oder NH ersetzt ist,
wobei Alk² jeweils unabhängig voneinander steht
für eine geradkettige oder verzweigte C₁- bis C₁₂-Alkylgruppe, die durch zumindest ein O unterbrochen sein kann,
wobei R⁶ steht für Alk¹,
wobei R⁷ und R⁸ jeweils unabhängig voneinander stehen
für Alk¹ oder Arl¹
wobei Eth¹ jeweils unabhängig voneinander steht
für -(CH₂-CH₂-O)ₘ₁-Alk² mit 1 ≤ m1 ≤ 16,
wobei Hal steht für F, Cl, Br oder I,
wobei Kt⁺ steht für Li⁺, Na⁺ oder K⁺,
und/oder deren Salz als Beschleuniger für eine photoinduzierte Reaktion von 3-(4-tert-butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder Mischungen dieser Verbindungen zu Avobenzon.

Vorteilhaft kann durch die Verwendung von zumindest einer Verbindung nach Formel I aufgrund der Beschleunigung der photoinduzierten Reaktion von 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischung der beiden Verbindungen zu Avobenzon der Verlust an UV-Absorptionsfähigkeit einer Zubereitung oder eines Materials zumindest teilweise ausgeglichen werden, da aufgrund der photoinduzierten Reaktion bei Bestrahlung Avobenzon und somit ein bekannter UV-Filter gebildet wird.

Aufgrund des Einsatzes zumindest einer Verbindung nach Formel I als Beschleuniger für die photoinduzierte Reaktion ist dabei auch eine nahezu vollständige Umwandlung des 3-(4-tert-Butyl-phenyl)-3-hydroxy-1-(4-methoxy-phenyl)-propan-1-on, des 1-(4-tert-Butyl-phenyl)-3-hydroxy-3-(4-methoxy-phenyl)-propan-1-on oder der Mischungen dieser Verbindungen in Avobenzon innerhalb eines vorbestimmten Zeitraumes möglich.

Bevorzugt ist dieser vorbestimmte Zeitraum t aus dem Zeitintervall gewählt, das einer minimalen Erythemdosis (MED) von 0,5 bis 50 MED, besonders bevorzugt von 5,0 bis 30 MED und ganz besonders bevorzugt von 10 bis 20 MED entspricht. Dabei entspricht eine durch Sonnenstrahlung applizierte absolute UV-Dosis von 50kJ/m² in etwa der erythemgewichteten Dosis von 1 MED.

Dadurch ist durch den Einsatz von 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen und zumindest einer Verbindung nach Formel I ein variabler Sonnenschutz möglich, der bei ansteigender Bestrahlungsdauer mehr und mehr von dem UV-Filter Avobenzon durch Photokonversion von 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on, freisetzt.

Bevorzugt wird 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder die Mischungen dieser Verbindungen zusammen mit zumindest einer Verbindung der Formel I, wie zuvor beschrieben, in Kombination mit zumindest einem weiteren UV-Filter verwendet. Bevorzugt weist der dementsprechende hinzukombinierte UV-Filter ein Absorptionsspektrum auf, das dem Absorptionsspektrum des Avobenzons zumindest hinsichtlich des Absorptionsmaximums von Avobenzon gleicht. Besonders bevorzugt wird als hinzukombinierter UV-Filter Avobenzon selbst verwendet. Ganz besonders bevorzugt ist es, wenn der hinzukombinierte UV-Filter von Avobenzon verschieden und zudem photostabil ist. In dieser Anordnung ist vor Bestrahlungsbeginn zunächst kein Avobenzon enthalten. Durch Photokonversion wird aber hier zusätzliche Absorptionskraft im Vergleich zum Ausgangswert aufgebaut. Beispiele für derartige als photostabil vermarktete hinzukombinierte UV-Filter sind beispielsweise 2-(4-Diethylamino-2-hydroxybenzoyl)-benzoesäurehexylester, vertrieben unter der Markenbezeichnungen Uvinul® A Plus der Firma BASF SE, 2,2'-Methylen-bis-(6-(2H-benzotriazol-2-yl)-4-(1,1,3,3-tetramethylbutyl)phenol), vertrieben unter der Bezeichnung Tinosorb® M der Firma BASF SE oder Salze der 2,2'-(1,4-Phenylen)bis)-1 H-benzimidazol-4,6-disulfonsäure), beispielsweise vertrieben unter der Bezeichnung Neo Heliopan® AP der Firma Symrise, 2-(2H-Benzotriazol-2-yl)-4-methyl-6-(2-methyl-3-(1,3,3,3-tetramethyl-1-(trimethylsilyloxy)disiloxanyl)propyl)phenol (INCI Drometriazole Trisiloxane), beispielsweise bekannt unter dem Handelsnamen Mexoryl XL der Firma l'Oréal oder 3,3'-(1,4-Phenylendimethylen)bis(7,7-dimethyl-2-oxobicyclo-[2.2.1]hept-1-yl-methansulfonsäure (INCI Terephthalidene Dicamphor Sulfonic Acid), beispielsweise bekannt unter dem Handelsnamen Mexoryl SX der Firma l'Oréal.

Somit kann durch die Verwendung von 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on, zusammen mit zumindest einer Verbindung der Formel I, wie zuvor beschrieben, entweder die photoinduzierte Zersetzung eines solchen hinzukombinierten UV-Filters hinsichtlich der absinkenden UV-Absorptionsfähigkeit mittels der Bildung von Avobenzon zumindest teilweise kompensiert werden, oder gegebenenfalls sogar zusätzliche Absorptionskraft generiert werden.

Bevorzugt wird eine Mischung aus zumindest einer Verbindung nach Formel I zusammen mit 3-(4-tert-Butyl-phenyl)-3-hydroxy-1-(4-methoxyphenyl)-propan-1-on, 1-(4-tert-Butyl-phenyl)-3-hydroxy-3-(4-methoxyphenyl)-propan-1-on oder der Mischungen dieser Verbindungen, insbesondere von 3-(4-tert-Butyl-phenyl)-3-hydroxy-1-(4-methoxy-phenyl)-propan-1-on, als photoinduzierter Stabilisator für einen UV-Filter verwendet. Besonders bevorzugt weist dabei der zu stabilisierende UV-Filter ein Energieniveau eines ersten angeregten Triplett-Zustandes auf, das mit einer Abweichung von maximal fünf Prozent dem Energieniveau des ersten angeregten Triplett-Zustandes von 3-(4-tert-Butyl-phenyl)-3-hydroxy-1-(4-methoxy-phenyl)-propan-1-on oder 1-(4-tert-Butyl-phenyl)-3-hydroxy-3-(4-methoxy-phenyl)-propan-1-on entspricht. Besonders bevorzugt wird die zuvor beschriebene Mischung als photoinduzierter Stabilisator für Avobenzon verwendet.

Durch Bestrahlung von Molekülen mit UV-Strahlen können dieselben die Strahlungsenergie absorbieren. Üblicherweise werden die Moleküle dabei von einem Grundzustand So in einen angeregten ersten Singulettzustand S₁ oder einen höheren Singulettzustand Sₓ angehoben. Aus einem solchen Singulettzustand können die Moleküle durch "Intersystem Crossing (ISC)" in einen Triplettzustand wechseln, und von diesem aus durch z.B. thermische Relaxation oder Strahlungsrelaxation in den Grundzustand zurückfallen. Somit ist es möglich, dass ein solches Molekül durch Absorption von UV-Strahlen von einem Grundzustand S₀ in einen ersten Singulettzustand S₁ angeregt wird, wobei es nachfolgend durch "Intersystem Crossing (ISC)" in den ersten angeregten Triplettzustand T₁ wechselt und dann durch Relaxationsprozesse wieder in den Grundzustand S₀ zurückfallen kann.

Bevorzugt wird eine Mischung aus zumindest einer Verbindung nach Formel I zusammen mit 3-(4-tert-Butyl-phenyl)-3-hydroxy-1-(4-methoxyphenyl)-propan-1-on, 1-(4-tert-Butyl-phenyl)-3-hydroxy-3-(4-methoxyphenyl)-propan-1-on oder der Mischungen dieser Verbindungen, insbesondere von 3-(4-tert-Butyl-phenyl)-3-hydroxy-1-(4-methoxy-phenyl)-propan-1-on, und zumindest einem weiteren UV-Filter als UV-Filtersystem für eine Zubereitung verwendet, wobei sich die Zubereitung mit einem solchen UV-Filtersystem in einem vorbestimmten Zeitraum durch eine im Wesentlichen gleichbleibende oder ansteigende UV-Absorptionsfähigkeit auszeichnet. Beispiele für einen weiteren UV-Filter neben der mindestens einen Verbindung der Formel I, wie zuvor beschrieben, sind nachfolgend gelistet.

Durch eine derartig gleichbleibende oder ansteigende UV-Absorptionsfähigkeit eines solchen UV-Filtersystems ist ein Sonnenschutz möglich, der der Haut oder dem jeweiligen, mit einem solchen UV-Filtersystem versehenen Material einen Schutz gewährleistet, der über den vorbestimmten Zeitraum zumindest eine gleichbleibende UV-Absorptionsfähigkeit sicherstellt.

Besonders im Falle der Haut ist eine ansteigende UV-Absorptionsfähigkeit besonders gewünscht, da mit fortschreitender Einwirkung der Strahlung auf die Haut dieselbe vermehrt gereizt wird. Somit benötigt die Haut mit fortschreitender Strahlungseinwirkung auch einen höheren Schutz, was vorteilhaft durch einen solchen Sonnenschutz mit einem derartigen UV-Filtersystem ermöglicht ist. Ein solches UV-Filtersystem ist in der Lage, den Schutz der Haut mit fortschreitender Reizung zu intensivieren. Somit bekommt die Haut den Schutz, den sie benötigt im Vergleich zu herkömmlichen Sonnenschutzzubereitungen, die in der Regel eine absinkende UV-Absorptionsfähigkeit bei fortschreitender Strahlungseinwirkung aufzeigen.

Ein weiterer Kerngedanke der Erfindung ist die Verwendung einer Mischung aus zumindest einer Verbindung nach Formel I, wie zuvor beschrieben, zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on; als photoinduziert-antioxidatives System. Dies ist insbesondere eine nicht-therapeutische Verwendung.

Die photoinduzierte Reaktion von 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on, zu Avobenzon zeichnet sich rein formal dadurch aus, dass im Zuge der Umwandlung einer der Verbindungen, wie zuvor beschrieben, zu Avobenzon, Wasserstoffequivalente abgegeben werden. Die Wasserstoffequivalente werden in der Regel nicht freigesetzt, sondern durch umgebende Verbindungen aufgenommen. Daher wirkt eine Mischung aus 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on, und Avobenzon bei Bestrahlung antioxidativ und somit photo-antioxidativ.

Somit ist unter einem photoinduziert-antioxidativen System eine Mischung aus 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on, und zumindest einer Verbindung nach Formel I, wie zuvor beschrieben, zu verstehen, die bei Bestrahlung eine antioxidative Wirkung aufzeigt.

Ein weiterer Gegenstand der Erfindung ist eine Zubereitung, enthaltend zumindest einen für kosmetische, pharmazeutische, dermatologische Zubereitungen, Nahrungsmittel, Nahrungsergänzungsmittel, Haushaltsprodukte oder Kunststoffe geeigneten Träger und eine Mischung aus zumindest einer Verbindung nach Formel I, wie zuvor beschrieben und 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere von 3-(4-tert-Butyl-phenyl)-3-hydroxy-1-(4-methoxy-phenyl)-propan-1-on und gegebenenfalls einen weiteren UV-Filter. Besonders bevorzugt weist der gegebenenfalls weitere UV-Filter oder mit anderen Worten der hinzukombinierte UV-Filter ein Absorptionsspektrum auf, das hinsichtlich des Absorptionsmaximums dem Absorptionsspektrum von Avobenzon gleicht. Ganz besonders bevorzugt ist es, wenn der hinzukombinierte UV-Filter von Avobenzon verschieden und zudem photostabil ist, wie zuvor beschrieben. Beispiele für derartige als photostabil vermarktete hinzukombinierte UV-Filter sind beispielsweise 2-(4-Diethylamino-2-hydroxybenzoyl)-benzoesäurehexylester, vertrieben unter der Markenbezeichnungen Uvinul® A Plus der Firma BASF SE, , 2,2'-Methylen-bis-(6-(2H-benzotriazol-2-yl)-4-(1,1,3,3-tetramethylbutyl)phenol), vertrieben unter der Bezeichnung Tinosorb® M der Firma BASF SE oder Salze der 2,2'-(1,4-Phenylen)bis)-1H-benzimidazol-4,6-disulfonsäure), beispielsweise vertrieben unter der Bezeichnung Neo Heliopan® AP der Firma Symrise, 2-(2H-Benzotriazol-2-yl)-4-methyl-6-(2-methyl-3-(1,3,3,3-tetramethyl-1-(trimethylsilyloxy)disiloxanyl)propyl)phenol (INCI Drometriazole Trisiloxane), beispielsweise bekannt unter dem Handelsnamen Mexoryl XL der Firma l'Oréal oder 3,3'-(1,4-Phenylendimethylen)bis(7,7-dimethyl-2-oxobicyclo-[2.2.1]hept-1-yl-methansulfonsäure (INCI Terephthalidene Dicamphor Sulfonic Acid), beispielsweise bekannt unter dem Handelsnamen Mexoryl SX der Firma l'Oréal.

Damit die Anzahl der UV-Filter in einer Zubereitung gering gehalten werden kann, ist es in einer Ausführungsform besonders bevorzugt, wenn der hinzukombinierte UV-Filter in der erfindungsgemäßen Mischung Avobenzon ist.

Bevorzugt weist eine erfindungsgemäße Zubereitung, wie zuvor beschrieben, während Bestrahlung mit Sonnen- oder sonnenähnlichem Licht innerhalb eines vorbestimmten Zeitraums maximal eine um +/-5 % abweichende UV-Absorptionsfähigkeit auf.

Unter sonnenähnlichem Licht versteht man eine Strahlung in einer spektralen Zusammensetzung analog der Sonnenstrahlung bzw. des Sonnenlichtes. Derartiges sonnenähnliches Licht kann zum Beispiel durch einen Solarsimulator erzeugt werden oder in Solarien zum Einsatz kommen, wobei die spektrale Zusammensetzung der Solarienstrahlung dem Sonnenlicht nicht exakt gleicht. Demzufolge soll unter sonnenähnlichem Licht auch Licht verstanden werden, das zumindest teilweise der spektralen Zusammensetzung des Sonnenlichtes gleicht oder das eine sonnenlichtähnliche Wirkung entfaltet.

Besonders bevorzugt weist eine solche Zubereitung während der Bestrahlung mit Sonnen- oder sonnenähnlichen Licht innerhalb eines vorbestimmten Zeitraums eine ansteigende UV-Absorptionsfähigkeit auf.

Ein weiterer Gegenstand der Erfindung ist ein Verfahren zur Stabilisierung der UV-Absorptionsfähigkeit einer Zubereitung mittels einer Mischung aus zumindest einer Verbindung nach Formel I, wie zuvor beschrieben, zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder der Mischungen dieser Verbindungen, insbesondere zusammen mit 3-(4-tert-Butyl-phenyl)-3-hydroxy-1-(4-methoxy-phenyl)-propan-1-on, wobei die Mischung und die Zubereitung so aufeinander abgestimmt sind, dass bei Bestrahlung mit Sonnen- oder sonnenähnlichem Licht die UV-Absorptionsfähigkeit der Zubereitung in einem vorbestimmten Zeitraum eine Abweichung von maximal +/- 5 Prozent aufweist oder ansteigt.

Bevorzugte Verbindungen der Formel I, die erfindungsgemäß verwendet werden, sind Verbindungen der Formel I, in denen X¹ und X² jeweils unabhängig voneinander für einen Substituenten der Formel II stehen und X³ für Alk¹ oder für einen Substituenten der Formel II steht.

Bei den Substituenten der Formel II ist es bevorzugt, wenn R² und R⁵ H bedeuten.

Bei den Substituenten der Formel II ist es bevorzugt, wenn R¹, R³ und R⁴ jeweils unabhängig voneinander stehen für H, OH, Alk¹, OAlk¹, Arl¹ Biphenylyl, COOAlk¹, CONHAlk¹, Het¹ oder einen Substituenten der Formel IV 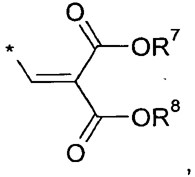 wobei R⁷ und R⁸ eine zuvor angegebene Bedeutung haben.

Steht X³ für Alk¹, so ist es bevorzugt, wenn Alk¹ Alk² bedeutet oder besonders bevorzugt Alk⁴ bedeutet. Ganz besonders bevorzugt ist Alk¹ in der Definition für X³ 2-Ethylhexyl.

Bei den jeweils unabhängigen Substituenten R¹, R³ und R⁴ in der Formel II ist es bevorzugt, wenn Alk¹ der Definition für Alk² oder Alk⁴ entspricht oder es ist ganz besonders bevorzugt, wenn Alk¹ Methyl oder 1,1-Dimethylpropyl bedeutet.

Bei den jeweils unabhängigen Substituenten R¹, R³ und R⁴ in der Formel II ist es bevorzugt, wenn OAlk¹ OAlk³ entspricht oder es ist ganz besonders bevorzugt, wenn OAlk¹ Methoxy, 2-Ethylhexyloxy oder O-CH₂-CH(OH)-CH₂-O-Isopropyl bedeutet.

Bei den jeweils unabhängigen Substituenten R¹, R³ und R⁴ in der Formel II ist es bevorzugt, wenn Arl¹ unsubstituiertes Phenyl bedeutet.

Bei den jeweils unabhängigen Substituenten R¹, R³ und R⁴ in der Formel II ist es bevorzugt, wenn COOAlk¹ COOAlk² bedeutet oder es ist ganz besonders bevorzugt, wenn COOAlk¹ 2-Ethylhexylcarboxy bedeutet.

Bei den jeweils unabhängigen Substituenten R¹, R³ und R⁴ in der Formel II ist es bevorzugt, wenn CONHAlk¹ CONHAlk² bedeutet oder es ist ganz besonders bevorzugt, wenn CONHAlk¹ tert.-Butylaminocarboxy bedeutet.

Bei den jeweils unabhängigen Substituenten R¹, R³ und R⁴ in der Formel II ist es bevorzugt, wenn die Substituenten R⁷ und R⁸ in Formel IV Alk⁴ entsprechen oder es ist ganz besonders bevorzugt, wenn R⁷ und R⁸ unabhängig voneinander Ethyl, Isobutyl ode Isopropyl entsprechen. Besonders bevorzugt sind R⁷ und R⁸ gleich.

Bei den jeweils unabhängigen Substituenten R¹, R³ und R⁴ in der Formel II ist es bevorzugt, wenn Het¹ bevorzugt 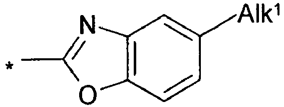 oder 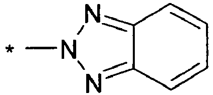 bedeutet. Ganz besonders bevorzugt ist Het¹

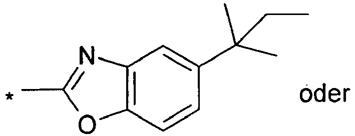

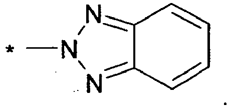

Besonders bevorzugte Verbindungen der Formel I sind Verbindungen, bei denen Y³-X³ ausgewählt wird aus der Gruppe der Kombinationen
a) Y³ steht für NH und X³ steht für Alk¹ oder für eine zuvor besonders bevorzugte Bedeutung für Alk¹,
b) Y³ steht für eine Einfachbindung und X³ steht für einen Substituenten der Formel II; wobei R¹, R², R⁴ und R⁵ H bedeuten und R³ OAlk¹, Arl¹ oder Biphenylyl bedeutet und wobei OAlk¹, Arl¹ eine der zuvor genannten bevorzugten Bedeutungen haben können oder
c) Y³ steht für NH und X³ steht für einen Substituenten der Formel II, wobei R¹ H oder Alk¹ bedeutet, R² und R⁵ H bedeuten, R⁴ H oder OH bedeutet und R³ COOAlk¹, CONHAlk¹, Het¹ oder einen Substituenten der Formel IV bedeutet, wobei Alk¹, COOAlk¹, CONHAlk¹, Het¹ oder der Substituent der Formel IV mit R⁷ und R⁸ eine der zuvor angegebenen Bedeutungen haben können.

Besonders bevorzugte Verbindungen der Formel I sind Verbindungen, bei denen Y¹-X¹ und Y²-X² jeweils unabhängig voneinander ausgewählt werden aus der Gruppe der Kombinationen
d) Y¹ und Y² stehen für eine Einfachbindung und X¹ und X² stehen jeweils unabhängig voneinander für einen Substituenten der Formel II, wobei R¹ H, OAlk¹ oder OH bedeutet, R², R⁴ und R⁵ H bedeuten und R³ OAlk¹, Arl¹ Alk¹ oder Biphenylyl bedeutet und wobei Alk¹, OAlk¹, Arl¹ eine der zuvor genannten bevorzugten Bedeutungen haben können oder
e) Y¹ und Y² stehen für NH und X¹ und X² stehen jeweils unabhängig voneinander für einen Substituenten der Formel II, wobei R¹ H oder OH bedeutet, R² und R⁵ H bedeuten, R⁴ H oder Alk¹ bedeutet und R³ COOAlk¹, Het¹ oder einen Substituenten der Formel IV bedeutet, wobei Alk¹, COOAlk¹, Het¹ oder der Substituent der Formel IV mit R⁷ und R⁸ eine der zuvor angegebenen Bedeutungen haben können.

Besonders bevorzugt sind Y¹-X¹ und Y²-X² gleich.

Weitere bevorzugte Kombinationen von Ausführungsformen sind in den Ansprüchen offenbart.

Erfindungsgemäß ganz besonders bevorzugt sind Verbindungen der Formeln X, XI, XII, XIII, XIV, XV, XVI, XVII, XVIII, XIX, XX, XXI oder XXII

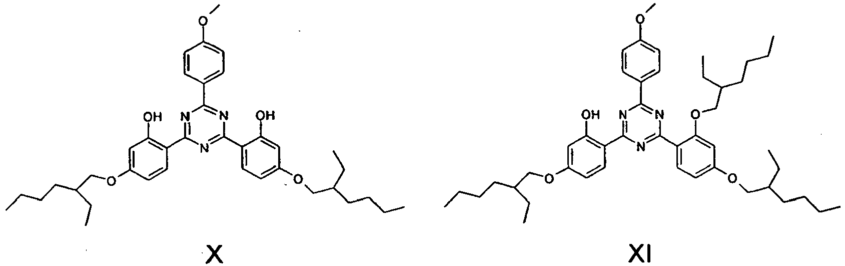

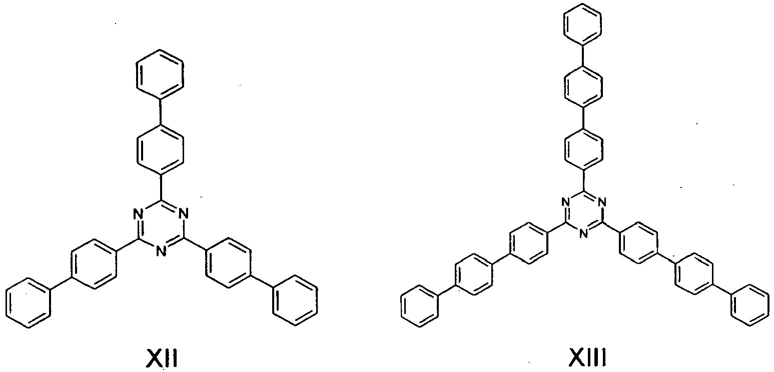

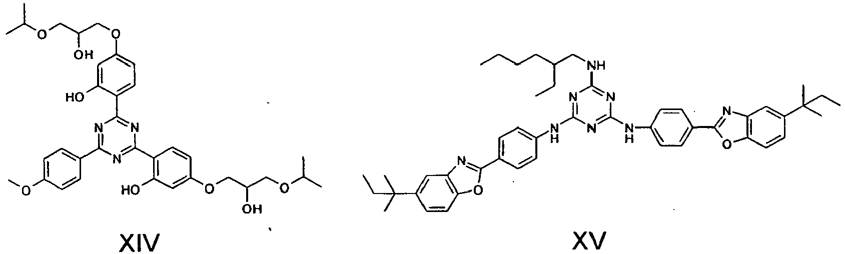

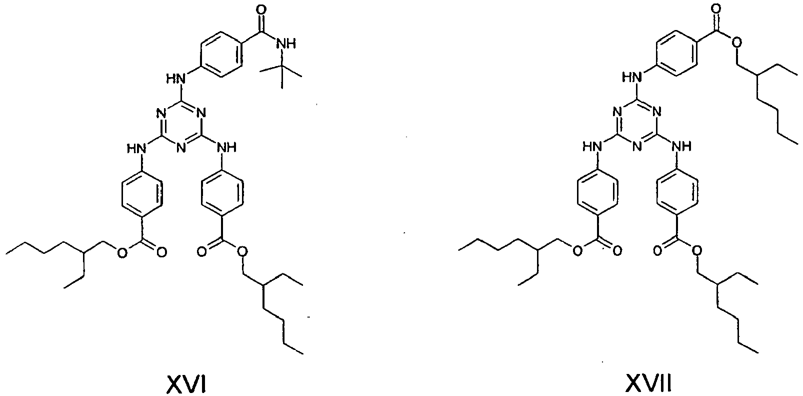

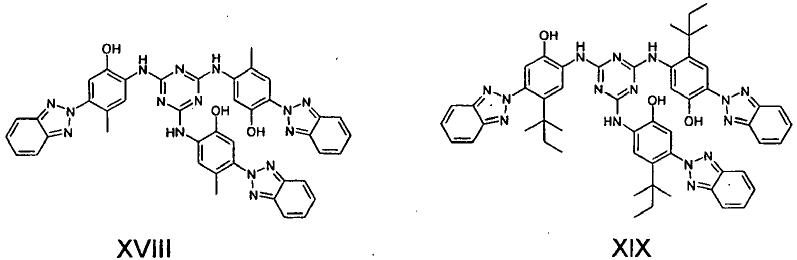

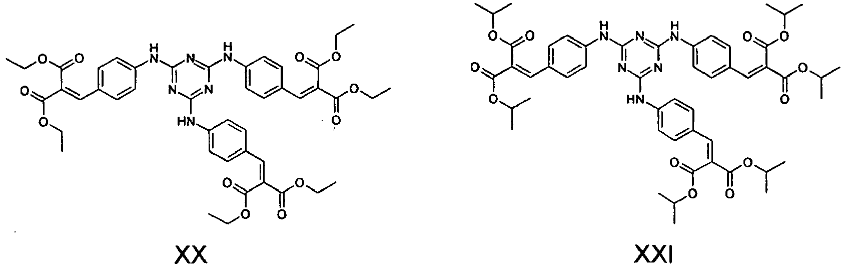

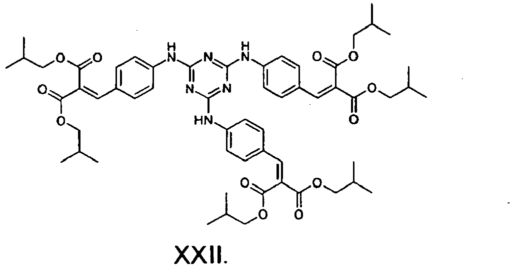

Aus dieser Gruppe der Einzelverbindungen stellvertretend für die Verbindungen der Formel I, sind insbesondere die Einzelverbindungen der Formel X, XII, XVI, XVII und XV bevorzugt in der erfindungsgemäßen Verwendung und in den erfindungsgemäßen Zubereitungen und Mischungen zu verwenden. Ganz besonders bevorzugt wird die Verbindung der Formel XVII ausgewählt.

Erfindungsgemäß sind ebenfalls Verbindungen der Formel I zu verwenden, die durch zumindest ein X¹ bis X³ an ein Polysiloxan angebunden sind.

Solche Triazine sind in der WO 2009/074409, in der WO 2009/053149 und in der WO 2006/128732 beschrieben und hiermit erfindungsgemäß offenbart.

Ein weiterer Gegenstand der vorliegenden Erfindung sind Zubereitungen, die 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder Mischungen dieser Verbindungen, insbesondere von 3-(4-tert-Butyl-phenyl)-3-hydroxy-1-(4-methoxy-phenyl)-propan-1-on, mit zumindest einer Verbindung der Formel I oder mit zumindest einer der zuvor beschriebenen oder als bevorzugt angegebenen Verbindungen nach Formel I oder die gelisteten Einzelverbindungen enthalten.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.
Bevorzugte Zubereitungen sind kosmetische Zubereitungen.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Gegenstand der Erfindung ist auch ein Verfahren zur Herstellung einer Zubereitung, wie zuvor beschrieben, wobei mindestens eine Verbindung nach Formel I zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder Mischungen dieser Verbindungen, insbesondere mit 3-(4-tert-Butyl-phenyl)-3-hydroxy-1-(4-methoxy-phenyl)-propan-1-on, und mit einem Träger und gegebenenfalls mit weiteren Aktiv- oder Hilfsstoffen vermischt wird. Geeignete Trägerstoffe sowie weitere UV-Filter oder Aktiv- oder Hilfsstoffe sind im nachfolgenden Teil ausführlich beschrieben.

In bevorzugten Ausführungsformen wird die mindestens eine Verbindung nach Formel I mit den definierten oder als bevorzugt angegebenen Substituenten oder bevorzugte Einzelverbindungen in den erfindungsgemäßen Zubereitungen typischerweise in Mengen von 0,05 bis 10 Gew.-%, bevorzugt in Mengen von 0,1 Gew.-% bis 5 Gew.-% und besonders bevorzugt in Mengen von 0,5 bis 2 Gew.-%, eingesetzt. Dabei bereitet es dem Fachmann keinerlei Schwierigkeiten die Mengen abhängig von der beabsichtigten Wirkung der Zubereitung entsprechend auszuwählen.

In den beschriebenen Zubereitungen, die erfindungsgemäß mindestens eine Verbindung nach Formel I mit den definierten oder als bevorzugt angegebenen Substituenten oder bevorzugte Einzelverbindungen und 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder Mischungen dieser Verbindungen, insbesondere 3-(4-tert-Butyl-phenyl)-3-hydroxy-1-(4-methoxy-phenyl)-propan-1-on, enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Bevorzugte Zubereitungen enthalten neben den Verbindungen nach Formel I, wie zuvor beschrieben oder als bevorzugt beschrieben, sowie den gegebenenfalls anderen Inhaltsstoffen weitere UV-Filter, beispielsweise organische UV-Filter. Dies sind beispielsweise die sogenannten hydrophilen oder lipophilen Sonnenschutzfilter, die im UVA-Bereich und/oder UVB-Bereich und(/oder IR und/oder VIS-Bereich (Absorber) wirksam sind. Diese Substanzen können insbesondere unter Zimtsäurederivaten, Salicylsäurederivaten, Campherderivaten, Triazinderivaten einer anderen Struktur als der eingesetzten Verbindung der Formel I in der Zubereitung, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere für eine Kombination geeignet sind:
para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Symrise, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Symrise.

β,β-Diphenylacrylate Derivate: Octocrylene, z. B. vetrieben unter dem Namen "Eusolex® OCR" von der Firma Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40"; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate oder 2-Hydroxy-4-methoxybenzophenon, vertrieben von der Fa. Merck, Darmstadt unter dem Namen Eusolex® 4360.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Symrise.

Phenylbenzotriazol Derivate: Drometrizole trisiloxane, z. B. vetrieben unter dem Namen "Silatrizole" von der Fa. Rhodia Chimie, Methylenebis(benzo-triazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vertrieben unter dem Namen "Tinosorb M" von der früheren Fa. BASF.

Anthranilin Derivate: Menthyl anthranilate, z. B. vetrieben unter dem Namen "Neo Heliopan MA" von der Fa. Symrise.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung 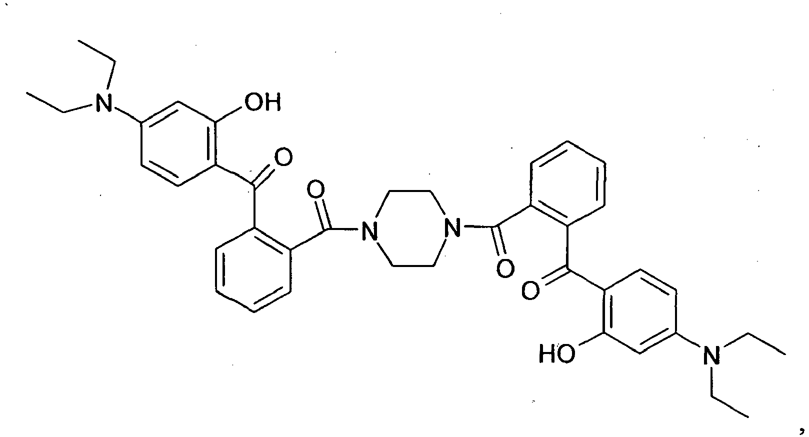 oder die UV-Filter der folgenden Strukturen 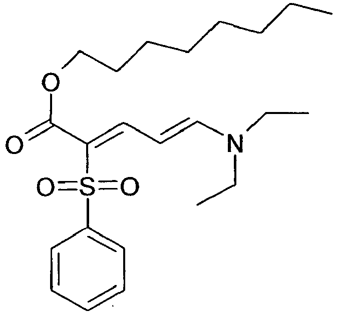 oder

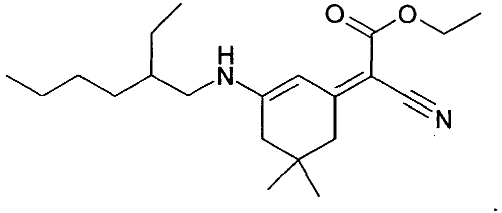

Bevorzugt kann auch mit UV-Filtern auf Basis von Polysiloxancopolymeren mit einer statistischen Verteilung gemäß nachfolgender Formel kombiniert werden, wobei z.B. a = 1,2; b= 58 und c=2,8 sind:

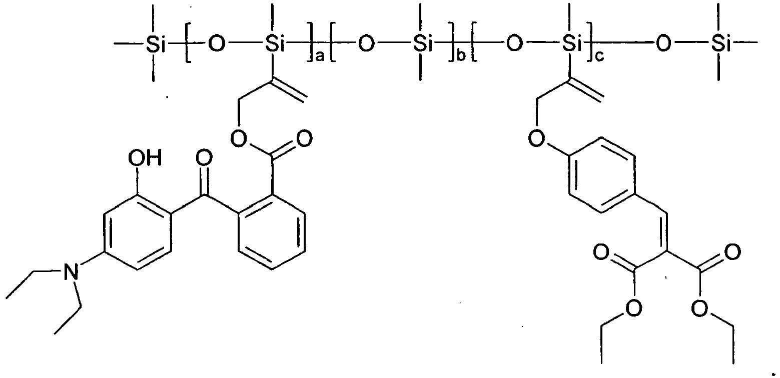

Die in der Liste aufgeführten Verbindungen sind nur als Beispiele aufzufassen. Selbstverständlich können auch andere UV-Filter verwendet werden.

Geeignete organischen UV-schützende Substanzen, die als weiterer UV-Filter in Frage kommen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene und Mischungen davon. Ganz besonders bevorzugte organische UV-Filter, die erfindungsgemäß hinzukombinierbar zu den Verbindungen der Formel I und 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder Mischungen dieser Verbindungen, insbesondere 3-(4-tert-Butyl-phenyl)-3-hydroxy-1-(4-methoxy-phenyl)-propan-1-on sind, wurden zuvor beschrieben.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Erfindungsgemäße Zubereitungen können neben den Verbindungen der Formel I und 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder Mischungen dieser Verbindungen, insbesondere 3-(4-tert-Butyl-phenyl)-3-hydroxy-1-(4-methoxy-phenyl)-propan-1-on anorganische UV-Filter enthalten, sogenannte partikuläre UV-Filter.

Diese Kombinationen mit partikulären UV-Filtern sind sowohl als Pulver als auch als Dispersion oder Paste der folgenden Typen möglich.

Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es bevorzugt sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminium-salze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
   o "Zinc Oxide CS-5" der Fa. Toshibi (ZnO nachbehandelt mit polymethylhydrogenosiloxane)
   o Nanogard Zinc Oxide FN der Fa. Nanophase Technologies
   o "SPD-Z1" der Fa Shin-Etsu (ZnO nachbehandelt mit einem Silikongepfropften Acrylpolymer, dispergiert in Cyclodimethylsiloxane
   ○ "Escalol Z100" der Fa ISP (Aluminiumoxid nachbehandeltes ZnO dispergiert in einer ethylhexyl methoxycinnamate/PVPhexadecene/methicone copolymer Mischung)
   o "Fuji ZNO-SMS-10" der Fa. Fuji Pigment (ZnO nachbehandelt mit Siliciumdioxid und Polymethylsilesquioxan);
   ○ Unbehandeltes Ceroxide Mikropigment z.B. mit der Bezeichnung "Colloidal Cerium Oxide" der Fa Rhone Poulenc
   o Unbehandelte und/oder nachbehandelte Eisenoxide mit der Bezeichnung Nanogar der Fa. Arnaud.

Beispielhaft können auch Mischungen verschiedener Metalloxide, wie z.B. Titandioxid und Ceroxid mit und ohne Nachbehandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid. Zinkoxid-Mischungen wie z.B. das Produkt UV-Titan M261 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Bevorzugte Zubereitungen können auch mindestens einen weiteren kosmetischen Wirkstoff enthalten, beispielsweise ausgewählt aus Antioxidantien, Anti-aging-Wirkstoffen, Anti-Cellulite Wirkstoffen, Selbstbräunungssubstanzen, hautaufhellenden Wirkstoffen oder Vitaminen.

Ferner sind erfindungsgemäße Kombinationen mit allen Wirkstoffen und Hilfstoffen kombinierbar, wie sie in WO2009/098139 systematisch gelistet sind. Insbesondere gehören diese Stoffe zu den darin aufgeführten Verwendungskategorien "Moisturizers and Humectants", "Desquamating agents", "Agents for improving the barrier function", "Depigmenting Agents", "Antioxidants", "Dermo-relaxing or dermo-decontracting agents", "Anti-glycation agents", "Agents for stimulating the synthesis of dermal and/or epidermal macromolecules and/or for preventing their degradation", "Agents for stimulating fibroblast or keratinocyte proliferation and/or keratinocyte differentiation", "Agents for promoting the maturation of the horny envelope", "NO-synthase inhibitors", "Peripheral benzodiazepine receptor (PBR) antagonists", "Agents for increasing the activity of the sebaceous glands", "Agents for stimulating the energy metabolism of cells", "Tensioning agents", "Fat-restructuring agents", "Sliming agents", "Agents for promoting the cutaneous microcirculation", "Calmatives or antiirritants", "Sebo-regulating or anti-seborrhoic agents", "Astringents", "Cicatrizing agents", "Anti-inflammatory agents", "Antiacne agents".

Die schützende Wirkung von Zubereitungen gegen oxidativen Stress bzw. gegen die Einwirkung von Radikalen kann zusätzlich verbessert werden, wenn die zuvor beschriebenen Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B 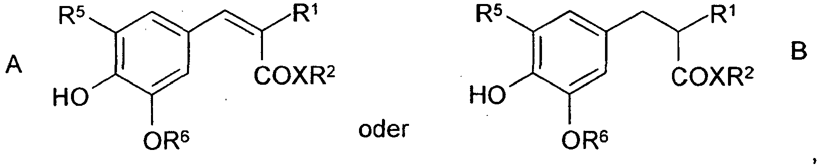 worin
R¹ aus der Gruppe -C(O)CH₃, -CO₂R³, -C(O)NH₂ und -C(O)N(R⁴)₂ ausgewählt werden kann,
X O oder NH,
R²lineares oder verzweigtes Alkyl mit 1 bis 30 C-Atomen,
R³lineares oder verzweigtes Alkyl mit 1 bis 20 C-Atomen,
R⁴jeweils ungabhängig voneinander H oder lineares oder verzweigtes Alkyl mit 1 bis 8 C-Atomen,
R⁵H oder lineares oder verzweigtes Alkyl mit 1 bis 8 C-Atomen oder lineares oder verzweigtes Alkoxy mit 1 bis 8 C-Atomen und
R⁶ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex^{®} ST Liquid) und/oder 2-(4-Hydroxy-3,5-dimethoxybenzyl)-malonsäure-bis-(2-ethylhexyl)ester (z.B. RonaCare^{®} AP). Ferner ist die Kombination mit 2-(4-Hydroxy-3-methoxybenzyliden)-malonsäure-bis-isopropylester bzw. 2-(4-Hydroxy-3-methoxybenzyl)-malonsäure-bis-isopropylester bevorzugt. Analoges gilt für entsprechende bis-Ethylester.

Die Bezeichnungen R¹ bis R⁶ und X gelten hier nur für die Reste der Formeln A und B und haben keinen Bezug zu den Resten der Formel I.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen, die erfindungsgemäß verwendet werden können, sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono-und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31(7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete anti-aging Wirkstoffe, insbesondere für hautpflegende Zubereitungen, sind vorzugsweise sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myoinositol-phosphat (DIP), cyclisches 2,3-diphosphoglycerat (cDPG), 1,1-Diglycerin-Phosphat (DGP), β-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Ferner können die erfindungsgemäßen Zubereitungen mindestens einen Selbstbräuner als weiteren Inhaltsstoff enthalten.

Als vorteilhafte Selbstbräuner können unter anderem eingesetzt werden: 1,3-Dihydroxyaceton, Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson). Ganz besonders bevorzugt ist das 1,3-Dihydroxyaceton, Erythrulose oder deren Kombination.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin oder Rucinol.

Die einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin- A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt. Ernährungsphysiologische Anwendungen orientieren sich am jeweiligen empfohlenen Vitaminbedarf.

Die beschriebenen Retinoide sind gleichzeitig auch wirksame Anti-Cellulite-Wirkstoffe. Ein ebenfalls bekannter Anti-Cellulite-Wrkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/V), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein. Für eine innerliche Anwendung sind Darreichungsformeln wie Kapseln, Dragees, Pulver, Tabletten-Lösungen oder Lösungen geeignet.

Als Anwendungsform der einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Die erfindungsgemäßen Zubereitungen können in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide).

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die einzusetzenden Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die kosmetischen und dermatologischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den bevorzugten O/W-Emulsionen zu verwenden.

Vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen.

Es ist ferner von Vorteil, die Fettsäureethoxylate ausfolgender Gruppe zu wählen:
Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol-(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)-glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen. Als fakultative, dennoch erfindungsgemäß gegebenenfalls vorteilhafte W/O-Emulgatoren können eingesetzt werden:
Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome,
Diglycerinester gesättigter und/oder ungesättigter, verzweigter und/oder unverzweigter Alkancarbonsäuren einer Kettenlänge von 8 bis 24,
insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen,
Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die öligalkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane..

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsformen der Erfindung sind in den Beispielen dargestellt und werden in der nachfolgenden Beschreibung näher erläutert, ohne den Umfang der vorliegenden Erfindung zu beschränken.

Es stellt dar:
- Bsp. 1: eine ansteigende UV-Absorbtionsfähigkeit von 3-(4-tert-Butyl-phenyl)-3-hydroxy-1-(4-methoxy-phenyl)-propan-1-on durch Zugabe von Uvinul® T150,
- Bsp. 2: mehrere beispielhafte kosmetische Zubereitungen enthaltend zumindest eine Verbindung nach Formel I und 3-(4-tert-Butyl-phenyl)-3-hydroxy-1-(4-methoxy-phenyl)-propan-1-on.

### Beispiel 1:

In einer Serie von Experimenten wurden nachfolgend genannte Lösungskompositionen mit einem Suntest CPS+ Gerät der Firma Atlas jeweils mit UV-Licht einer Dosis von 500kJ/m², entsprechend 10 MED (minimale erythemale Dosis), bestrahlt. Durch diese Bestrahlung wird 3-(4-tert-Butyl-phenyl)-3-hydroxy-1-(4-methoxy-phenyl)-propan-1-on (MeO-MBM) in Butylmethoxy-dibenzoylmethan (Avobenzon) konvertiert. Die Menge an gebildetem Avobenzon kann durch Auswertung der charakteristischen UV-Absorptionsbande bei 358nm bestimmt werden.

Als kosmetische Vehikel wurden Miglyol 812^{®} (Capry/Caprinsäure-Triglycerid) der Firma Sasol bzw. Arlasolv DMI^{®} (Dimethylisosorbid) von Croda eingesetzt. Uvinul T150^{®}, soweit verwendet, wurde von BASF bezogen. Als 100% Referenz fungiert dabei jeweils die Zubereitung ohne Uvinul T150^{®}.

| Lösungskomposition | Relativer Gehalt an Avobenzon vor Bestrahlung | Relativer Gehalt an Avobenzon nach Bestrahlung |
|---|---|---|
| MeO-MBM (2w%) in Miglyol 812^{®} | 0% | 100% |
| MeO-MBM (2w%) und Uvinul T150 (1w%) in Miglyol 812^{®} | 0% | 156% |
| MeO-MBM (2w%) in Arlasolv DMI | 0% | 100% |
| MeO-MBM (2w%) und Uvinul T150 (1w%) in Arlasolv DMI | 0% | 178% |

Es zeigte sich, dass durch erfindungsgemäße Kombination von MeO-MBM und Uvinul T150 in verschiedenen Ölen die Bildung von Avobenzon durch Photokonversion von MeO-MBM unerwartet beschleunigt wurde.

### Zubereitungsbeispiele:

Nachfolgend aufgeführte Zubereitungbeispiele können desweiteren fogende Stabilisatoren enthalten (je 0,1-10% in Einzelkomponente oder in Gemischen): Benzotriazolyl Dodecyl p-Cresol (Tinoguard TL), Butyloctyl salicylate, Diethylhexyl 2,6-Naphthalate, Diethylhexyl Syringylidene malonate, Polyester-8 (Polycrylene), bis-Ethylhexyl Hydroxydimethoxy Benzylmalonate.

### Beispiel 1: W/O Emulsion

| | 54-07-5-A | 54-07-5-B | 54-07-5-C | 54-07-5-D | 54-07-5-E |
|---|---|---|---|---|---|
| Cetyl PEG/PPG-10/1 dimethicone (Abil EM 90) | 3,00 | 3,00 | 3,00 | 3,00 | 3,00 |
| Polyglyceryl-4 isostearate (Isolan GI 34) | 1,50 | 1,50 | 1,50 | 1,50 | 1,50 |
| Butylphthalimide isopropylphthalimide (Pelemol^{®} BIP) | 5,00 | 5,00 | 5,00 | 5,00 | 5,00 |
| Dimethyl isosorbide (Arlasolve DMI) | 5,00 | 5,00 | 5,00 | 5,00 | 5,00 |
| 3-(4-tert-Butyl-phenyl)-3-hydroxy-1-(4-methoxy-phenyl)-propan-1-on | 1,00 | | | 1,00 | 2,00 |
| 1-(4-tert-Butylphenyl)-3-hydroxy-3-(4-methoxy-phenyl)-propan-1-on | 2,00 | 1,00 | 3,00 | | |
| Uvinul^{®} A Plus | | 0,84 | 0,84 | 1,00 | |
| Formel XVI (Uvasorb Heb) | 2,0 | 2,0 | 1,5 | 4,0 | 5,0 |
| Formel X (Tinosorb S) | 2,0 | 2,0 | 3,0 | 1,5 | |
| Ascorbinsäure | | | 0,37 | 1,00 | 3,00 |
| Mineral Oil | 8,00 | 8,00 | 8,00 | 8,00 | 8,00 |
| Ethylhexyl stearate (Tegosoft^{®} OS) | 5,00 | 5,00 | 5,00 | 5,00 | 5,00 |
| Cyclomethicone (and) Aluminium/Magnesium Hydroxide Stearate (Gilugel SIL 5) | 5,00 | 5,00 | 5,00 | 5,00 | 5,00 |
| Preservative | 1,00 | 1,00 | 1,00 | 1,00 | 1,00 |
| Water | Ad 100 | Ad 100 | Ad 100 | Ad 100 | Ad 100 |
| NaCl | 0,50 | 0,50 | 0,50 | 0,50 | 0,50 |
| EDTA | 0,10 | 0,10 | 0,10 | 0,10 | 0,10 |
| Citronensäure q.S. | | | | | |

Herstellung: Pelemol^{®} BIP, Arlasolv DMI und Emulgatoren werden vorgelegt. 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on, Uvinul^{®} A Plus und die Trazine werden darin gelöst. Die restlichen Bestandteile der Ölphase werden zugegeben und homogen vermischt. Unter Rühren wird die auf pH=4-5 eingestellte Wasserphase einemulgiert. Anschließend wird homogenisiert. Die Emulsionen können unter schonenden Bedingungen bei Raumtemperatur hergestellt werden.

### Beispiel II: Wasserfestes Sonnenschutzspray

| A | | | |
|---|---|---|---|
| 3-(4-tert-Butyl-phenyl)-3-hydroxy-1-(4-methoxy-phenyl)-propan-1-on | 1,00 | 1,00 | 2,00 |
| 1-(4-tert-Butyl-phenyl)-3-hydroxy-3-(4-methoxy-phenyl)-propan-1-on | | 2,00 | 1,50 |
| Diethylhexyl Syringylidenemalonate, Caprylic/Capric Triglyceride (Oxynex^{®} ST Liquid) | | 0,50 | |
| RonaCare^{®} AP | | 2,00 | |
| Formel X (Tinsorb S) | 1,5 | 1,0 | 1,5 |
| Formel XVI (Uvasorb Heb) | 1,5 | 3,0 | 5,0 |
| Formel XVII (Uvinul T150) | 1,5 | 0,5 | |
| Ascorbyl Palmitate | | | 1,00 |
| Cyprylic/capric Triglyceride (Miglyol 812 N) | 7,00 | 7,00 | 7,00 |
| Butylphthalimide isopropylghthalimide (Pelemol^{®} BIP) | 10,00 | 10,00 | 10,00 |
| C12-15 alkyl benzoate (Tegosoft^{®} TN) | 10,00 | 10,00 | 10,00 |
| Phenethyl benzoate | 5,00 | 5,00 | 5,00 |
| (X-Tend 226) | | | |
| RonaCare^{®} Tocopherolacetat | 1,00 | 1,00 | 1,00 |

| B | | | |
|---|---|---|---|
| Cyclopentasiloxane (Dow Corning 245) | 43,80 | 41,30 | 41,80 |
| Phenyltrimethicone (Dow Corning 556) | 2,00 | 2,00 | 2,00 |
| Cyclopentasiloxane, dimethiconol Dow Corning 1501 Fluid | 20,00 | 20,00 | 20,00 |
| Parfümöl (q.s.) | 0,20 | 0,20 | 0,20 |

Herstellung: Die Komponenten der Phase A werden bei Raumtemperatur zusammengefügt und gerührt. Anschließend wird Phase B gemischt und unter Rühren zu Phase B gegeben und gerührt.

### Beispiel III: Pump Haarspray

| A | | | |
|---|---|---|---|
| 3-(4-tert-Butyl-phenyl)-3-hydroxy-1-(4-methoxy-phenyl)-propan-1-on | 1,00 | 2,00 | 4,00 |
| Formel XII | 1,00 | 1,00 | 1,00 |
| Formel X | | 1,00 | |
| 1-(4-tert-Buty(-phenyl)-3-hydroxy-3-(4-methoxy-phenyl)-propan-1-on | 1,00 | | 1,50 |
| Ethanol 96% reinst | Ad 100 | Ad 100 | Ad 100 |
| PVPNA copolymer PVP/VA W 735 | 6,00 | 6,00 | 6,00 |

| B | | | |
|---|---|---|---|
| Diethylhexyl Syringylidenemalonate, Caprylic/Capric Triglyceride (Oxynex^{®} ST Liquid) | 0,06 | 0,25 | 0,50 |
| PEG-75 Lanolin BHT (Solan E - Low Dioxane) | 0,20 | 0,20 | 0,20 |
| Parfum (Frag 280853 Green Activating) | 0,10 | 0,10 | 0,10 |

| C | | | |
|---|---|---|---|
| Wasser, demineralisiert | 13,00 | 13,00 | 13,00 |
| Titriplex III | 0,10 | 0,10 | 0,10 |
| PEG-12 dimethicone Dow Corning 193 Fluid | 0,50 | 0,50 | 0,50 |
| 0,1% D&C Red No 33 (CI 17200) in Wasser | 0,20 | 0,20 | 0,20 |
| PEG-40 Hydrogenated Castor Oil (Cremophor RH 410) | 1,00 | 1,00 | 1,00 |

Herstellung: Phase A vorlösen. Unter Rühren Phase B zu Phase A geben. Phase C vormischen und zum Rest geben, rühren, bis eine homogene Mischung entstanden ist.

### Beispiel IV: W/O-Emulsionen

| **Emulsion** | **A*** | **B** | **C** | **D** | **E** | **F*** |
|---|---|---|---|---|---|---|
| Polyglyceryl-2-Dipolyhydroxystearat | 3 | 5 | 3 | | | |
| PEG-30 Dipolyhydroxystearat | | | 2 | 3 | 4 | 5 |
| Natrium-Stärke Octenylsuccinat | 0,5 | 0,4 | | 0,3 | | 1 |
| Glycin | 0,3 | 0,3 | 0,5 | 0,4 | | |
| Alkohol | | 5 | 2 | 5 | 4 | |
| Magnesiumsulfat | 0,2 | 0,3 | 0,3 | 0,4 | 0,5 | 0,2 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylenglykol | | | 1 | | 5 | 3 |
| Glycerin | 3 | 5 | 7 | 10 | 3 | 3 |
| C18-38 Säuretriglyceride | 0,5 | | 1 | | 1 | |
| Titandioxid | 5 | 6 | 4 | | | 4 |
| Zinkoxid | 5 | | | | | |

| | | | | | | |
|---|---|---|---|---|---|---|
| * Vergleichsbeispiel | | | | | | |

| **Emulsion** | **A** | **B** | **C** | **D** | **E** | **F** |
|---|---|---|---|---|---|---|
| Formel X (Tinsosorb S) | | 3 | 3 | 2 | | |
| Formel XVII (Uvinul T150) | | 4,5 | 3 | | 3 | |
| 3-(4-tert-Butyl-phenyl)-3-hydroxy-1-(4-methoxyphenyl)-propan-1-on | 2,0 | 0,5 | 1,0 | 1,0 | 3,0 | |
| 1-(4-tert-Butyl-phenyl)-3-hydroxy-3-(4-methoxy-phenyl)-propan-1-on | | 1,5 | 1,0 | | | 3,0 |
| Formel XVI (Uvasorb Heb) | | | 1,5 | 4 | | |
| Butyl Methoxydibenzoylmethan | 2 | 3 | 4 | | 1 | 3 |
| Uvinul^{®} A Plus | | | | 4 | 2 | |
| Ethylhexylmethoxycinnamat | | | | | 7 | 5 |
| Benzotriazol an Gelatine gekoppelt | 4 | | 6 | | | |
| Taurin | 0,1 | | | 0,5 | 0,2 | |
| Vitamin E Acetat | 0,2 | 02 | | 0,3 | 0,1 | 0,5 |
| Na₂H₂EDTA | 0,1 | 0,1 | 0,2 | 0,2 | 0,2 | 0,5 |
| C8-C16 Alkylpolyglycosid | 1 | | | | | |
| Parfüm, Konservierungsmittel | q.s. | q.s | q.s | q.s | qs. | qs. |
| Farbstoffe, usw. | q.s. | q.s. | q.s. | q.s | q.s. | q.s. |
| Natriumhydroxid | q.s. | q.s. | q.s. | q.s | q.s. | q.s. |
| Wasser | ad 100,0 | ad 100,0 | ad 100,0 | ad 100,0 | ad 100,0 | ad 100,0 |

### Beispiel V: Haarpflegeformulierung

| **Gehalt in g Komponente per 100 g Formulierung** | | | | | | |
|---|---|---|---|---|---|---|
| **Komponente** | **A** | **B** | **C** | **D** | **E** | **F** |
| Disodium EDTA | 0.100 | 0.100 | 0.100 | 0.100 | 0.100 | 0.100 |
| Oxynex^{®}ST | 2.000 | 2.000 | 2.000 | 2.000 | 2.000 | 2.000 |
| 3-(4-tert-Butyl-phenyl)-3-hydroxyl-(4-methoxy-phenyl)-propan-1-on | 1,5 | 0,25 | 0,50 | 1,50 | 2,00 | 4,00 |
| 1-(4-tert-Butyl-phenyl)-3-hydroxy-3-(4-methoxyphenyl)-propan-1-on | | 1,75 | 2,5 | | | |
| Formel XVII (Uvinul T150) | 0,50 | 0,50 | 0,50 | 0,50 | 0,50 | 0,50 |
| Formel XII | 0,50 | 0,50 | 0,50 | 0,50 | 0,50 | 0,50 |
| Hexamidine diisethionate | 0.100 | 0 | 0 | 0 | 0 | 0 |
| Tetrahydrocurcumin | 0 | 0.500 | 0 | 0 | 0 | 0 |
| Glycyrrhetinic acid | 0 | 0 | 0.300 | 0 | 0 | 0 |
| Thiotaine®¹ | 0 | 0 | 0 | 5.000 | 0 | 0 |
| N-undecylenoyl-L-phenylalanine | 0 | 0 | 0 | 0 | 1.000 | 0 |
| N-acetyl glucosamine | 0 | 0 | 0 | 0 | 0 | 2.000 |
| Niacinamide | 5.000 | 5.000 | 5.000 | 5.000 | 5.000 | 5.000 |
| Citric acid | 0.015 | 0 | 0 | 0 | 0 | 0 |
| Isohexadecane | 3.000 | 3.000 | 3.000 | 3.000 | 3.000 | 3.000 |
| Isopropyl isostearate | 1.330 | 1.330 | 1.330 | 1.330 | 1.330 | 1.330 |
| Isopropyl N-laurosylsarcosinate | 0 | 0 | 5.000 | 0 | 0 | 0 |
| Sucrose polycottonseedate | 0.670 | 0.670 | 0.670 | 0.670 | 0.670 | 0.670 |
| Polymethylsilsesquioxane | 0.250 | 0.250 | 0.250 | 0.250 | 0.250 | 0.250 |
| Cetearyl glucoside + cetearyl alcohol | 0.200 | 0.200 | 0.200 | 0.200 | 0.200 | 0.200 |
| Behenyl alcohol | 0.400 | 0.400 | 0.400 | 0.400 | 0.400 | 0.400 |
| Ethylparaben | 0.200 | 0.200 | 0.200 | 0.200 | 0.200 | 0.200 |
| Propylparaben | 0.100 | 0.100 | 0.100 | 0.100 | 0.100 | 0.100 |
| Cetyl alcohol | 0.320 | 0.320 | 0.320 | 0.320 | 0.320 | 0.320 |
| Stearyl alcohol | 0.480 | 0.480 | 0.480 | 0.480 | 0.480 | 0.480 |
| Tocopheryl acetate | 0.500 | 0.500 | 0.500 | 0.500 | 0.500 | 0.500 |
| PEG-100 stearate | 0.100 | 0.100 | 0.100 | 0.100 | 0.100 | 0.100 |
| Glycerin | 7.000 | 7.000 | 7.000 | 7.000 | 7.000 | 7.000 |
| Titanium dioxide | 0.604 | 0.604 | 0.604 | 0.604 | 0.604 | 0.604 |
| Polyacrylamide + C13-14 isoparaffin + laureth-7 | 3.000 | 2.000 | 2.000 | 2.000 | 2.000 | 2.000 |
| Panthenol | 1.000 | 1.000 | 1.000 | 1.000 | 1.000 | 1.000 |
| Benzyl alcohol | 0.400 | 0.400 | 0.400 | 0.400 | 0.400 | 0.400 |
| Dimethicone + dimethiconol | 2.000 | 2.000 | 2.000 | 2.000 | 2.000 | 2.000 |
| Water (to 100 g) | to 100 | to 100 | to 100 | to 100 | to 100 | to 100 |
| TOTAL | 100 | 100 | 100 | 100 | 100 | 100 |

### Beispiel VI: Haarpflegeformulierung

| **Gehalt in g Komponente per 100 g Formulierung** | | | |
|---|---|---|---|
| **Komponente** | **G** | **H** | **I** |
| Disodium EDTA | 0.100 | 0.100 | 0.100 |
| Oxynex^{®} ST | 2.000 | 2.000 | 2.000 |
| Formel X (Tinsorb S) | 1,00 | 1,00 | 1,00 |
| Formel XVII (Uvinul T150) | 1,00 | 1,00 | 1,00 |
| 3-(4-tert-Butyl-phenyl)-3-hydroxy-1-(4-methoxy-phenyl)-propan-1-on | | 3,50 | 1,50 |
| 1-(4-tert-Butyl-phenyl)-3-hydroxy-3-(4-methoxy-phenyl)-propan-1-on | 2,00 | | |
| Cetyl pyridinium chloride | 0.200 | 0 | 0 |
| Pitera^{®} | 0 | 10 | 0 |
| Ascorbyl glycoside | 0 | 0 | 2.000 |
| Niacinamide | 5.000 | 5.000 | 5.000 |
| Polyquaternium 37 | 0 | 0 | 0 |
| Isohexadecane | 3.000 | 3.000 | 3.000 |
| Isopropyl isostearate | 1.330 | 1.330 | 1.330 |
| Sucrose polycottonseedate | 0.670 | 0.670 | 0.670 |
| Polymethylsilsesquioxane | 0.250 | 0.250 | 0.250 |
| Cetearyl glucoside + cetearyl alcohol | 0.200 | 0.200 | 0.200 |
| Behenyl alcohol | 0.400 | 0.400 | 0.400 |
| Ethylparaben | 0.200 | 0.200 | 0.200 |
| Propylparaben | 0.100 | 0.100 | 0.100 |
| Cetyl alcohol | 0.320 | 0.320 | 0.320 |
| Stearyl alcohol | 0.480 | 0.480 | 0.480 |
| Tocopheryl acetate | 0.500 | 0.500 | 0.500 |
| PEG-100 stearate | 0.100 | 0.100 | 0.100 |
| Glycerin | 7.000 | 7.000 | 7.000 |
| Titanium dioxide | 0.604 | 0.604 | 0.604 |
| Polyacrylamide + C13-14 isoparaffin + laureth-7 | 2.000 | 2.000 | 2.000 |
| Panthenol | 1.000 | 1.000 | 1.000 |
| Benzyl alcohol | 0.400 | 0.400 | 0.400 |
| Dimethicone + dimethiconol | 2.000 | 2.000 | 2.000 |
| Water (to 100 g) | to 100 | to 100 | to 100 |
| TOTAL | 100 | 100 | 100 |

### Beispiel VII: O/W-Emulsionen

| **Emulsion** | **A** | **B** | **C** | **D** | **E** | **F** |
|---|---|---|---|---|---|---|
| Glyceryl Stearat Citrat | 2,5 | 2 | 3 | | | |
| Sorbitanstearat | 0,5 | | | 2 | 1,5 | 2 |
| Polyglyceryl-3 Methylglycose Distearat | | | | 2,5 | 3 | 3 |
| Polyglyceryl-2 Dipolyhydroxystearat | | 0,8 | | | | 0,5 |
| Cetearylalkohol | | | | 1 | | |
| Stearylalkohol | 2 | | | | | 2 |
| Cetylalkohol | | 1 | | | 3 | |
| Acrylates/C₁₀₋₃₀ Alkyl Acrylat Crosspolymer | | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | 5 | 3 | | | 5 | |
| C₁₂₋₁₃ Alkyl Tartrat | | 2 | | | | |
| Butylenglycol Dicaprylat/Dicaprat | 5 | | | | 3 | 3 |
| Dicaprylyl Ether | | | | | 2 | |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Propylengykol | | | 1 | | 5 | 3 |
| Glycerin | 3 | 5 | 7 | 10 | 3 | 3 |
| C18-C38 Säuretriglyceride | 0,5 | | 1 | | 1 | |
| Titandioxid | 5 | | | 2 | | |
| 2,2'-Methylen-bis-(6-(2H-benzotriazol-2-yl)-(1,1,3,3-tetramethylbutyl)phenol) | 2,5 | | | | | |
| Formel XII | | 2 | | | | |
| Formel XVI (Uvasorb Heb) | 4 | | 3 | | 3 | 2 |
| Benzotriazol an Gelatine gekoppelt | | 5 | | 10 | | 3 |
| C8-C16 Alkylpolyglycosid | 1 | 0,6 | | | | |
| Formel XV | | | 2 | | | |
| Uvinul^{®} A Plus | 2 | | | | | 1 |
| Homosalat | | 5 | | 1 | | |
| Phenylbenzimidazol Sulfonsäure | | | 2 | | | 1 |
| Benzophenon-3 | 2 | | | | 2 | |
| Octylsalicylat | 5 | 5 | | 2 | | |
| Octocrylen | 2 | | | | 3 | 1 |
| 3-(4-tert-Butyl-phenyl)-3-hydroxy-1-(4-methoxy-phenyl)-propan-1-on | | 2,0 | 3,0 | 1,0 | 2,0 | 3,0 |
| 1-(4-tert-Butyl-phenyl)-3-hydroxy-3-(4-methoxyphenyl)-propan-1-on | 3,0 | | | 2,0 | | |
| Formel X (Tinsosorb S) | 1,2 | 3 | 2 | 1 | | |
| Parsol^{®} SLX | | | 3 | | | |
| Dihydroxyacetat | | | | | 4 | |
| Taurin | 0,1 | | | 0,5 | 0,2 | |
| 8-Hexadecen-1,16-dicarbonsäure | | 0,2 | | | | |
| Vitamin E Acetat | 0,2 | 0,2 | | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100, 0 |

### Beispiel VIII: O/W-Emulsionen

| **Emulsion** | **G** | **H** | **I** | **K** | **L** | **M** |
|---|---|---|---|---|---|---|
| Ceteareth-20 | 1 | 1,5 | 1 | | | |
| Sorbitanstearat | | | 0,5 | | 0,5 | |
| Glyceryl Stearat SE | | | | 1 | 1 | 1,5 |
| Emulgade F^{®} | | | | 2,5 | 2,5 | 3 |
| Cetearylalkohol | | | | 1 | | |
| Stearylalkohol | | | | | 1,5 | |
| Cetylalkohol | | | 0,5 | | | 2 |
| Acrylates/C₁₀₋₃₀ Alkyl Acrylat Crosspolymer | 0,2 | 0,4 | 0,3 | 0,1 | | |
| Carbomer | | | | | 0,3 | |
| Xanthan Gum | | | | 0,4 | | 0,4 |
| C₁₂₋₁₅ Alkyl Benzoat | 5 | 3 | | | 5 | |
| 2-Phenylbenzoat | | 2 | | | | |
| Butylenglycol Dicaprylat/Dicaprat | 5 | | | | 3 | 2 |
| Dicaprylyl Ether | | | | | 2 | |
| Diethylhexylnaphthalat | 2 | | | | | |
| Dicaprylcaprat | | 2 | | | 2 | 2 |
| Cyclomethicon | 5 | | 5 | 10 | | |
| Isohexadecan | | | | 5 | | |
| Mineralöl | | 1 | | | | |
| Propylenglykol | | | 4 | | | |
| Glycerin | 5 | 7 | 3 | 5 | 6 | 8 |
| C18-38 Säuretriglyceride | 0,5 | | 1 | | 1 | |
| Titandioxid | 5 | | 3 | 2 | | |
| NeoHeliopan^{®} AP | | 2 | | | 1 | 1 |
| Phenylbenzimidazol Sulfonsäure | 1 | | | 1 | 2 | 1 |
| Ethylhexylmethoxycinnamat | 5 | | 4 | 4 | | |
| Formel XVII (Uvinul T150) | | 2 | | 1 | | |
| Formel XVI (Uvasorb Heb) | 1 | | | | 3 | |
| Butyl Methoxydibenzoylmethan | 2,5 | | 2 | 2 | | 1 |
| Formel X (Tinsosorb S) | 2 | 0,5 | 1,5 | | | 2,0 |
| 4-Methylbenzyliden Camphor | 3 | | | | | |
| Parsol® SLX | | | | | 2 | |
| 3-(4-tert-Butyl-phenyl)-3-hydroxy-1-(4-methoxy-phenyl)-propan-1-on | 1,0 | 2,0 | 4,0 | | 1,5 | 3,0 |
| 1-(4-tert-Butyl-phenyl)-3-hydroxy-3-(4-methoxy-phenyl)-propan-1-on | 1,0 | | | 5,0 | | |
| Kreatinin | 0,1 | 0,01 | 0,05 | | | |
| Kreatin | 0,5 | 0,2 | 0,1 | | | |
| Licorice Extrakt/Licochalkon | | | | 0,5 | | |
| Vitamin E Acetat | 0,2 | | | 0,5 | 0,5 | 0,5 |
| Tapioka Stärke | | 3 | | | 2 | |
| Na₂H₂EDTA | 0,1 | | 0,2 | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel IX: O/W-Emulsionen

| **Emulsion** | **N** | **O** | **P** | **Q** | **R** | **S** |
|---|---|---|---|---|---|---|
| Glycerylstearat SE | | 2 | | 2 | | |
| Glycerylstearat | 2 | | 2 | | | |
| PEG-40 Stearat | | | 2 | | 1 | |
| PEG-10 Stearat | | | | 2,5 | 1 | |
| Ceteareth-20 | | | | | | 2,6 |
| Natrium Cetyl Phosphate | | | | | 2 | |
| Glyceryl Stearat, Ceteareth-12, Ceteareth-20, Cetearyl Alcohol, Cetyl Palmitat | | | | | | 5,4 |
| Stearinsäure | 3 | 2 | | | 2 | |
| Stearylalkohol | | 2 | 2 | | | |
| Stearylalkohol | 0,5 | | 2 | | | |
| Cetylalkohol | 3 | | | 2 | | |
| Acrylates/C₁₀₋₃₀ Alkyl Acrylat Crosspolymer | | | 0,2 | | 0,4 | |
| Carbomer | | 0,3 | | 0,3 | 0,3 | |
| Xanthan Gum | | 0,3 | 0,4 | | | |
| C₁₂₋₁₅ Alkyl Benzoat | 5 | | | | 5 | 3 |
| 2-Phenylbenzoat | 5 | | | | | |
| Butylenglycol Dicaprylat/Dicaprat | | 5 | | 4 | | 3 |
| Dicaprylyl Ether | | 2 | | | 3 | |
| Diethylhexylnaphthalat | 3 | | | | | |
| Cyclomethicon | 2 | | 10 | 2 | | |
| Isohexadecan | | | | 2 | 3 | |
| Mineralöl | | | | | 3 | |
| Propandiol | | 3 | | 5 | | |
| Glycerin | 3 | 5 | 10 | 7 | 4 | 5 |
| Titandioxid | 2 | 4 | | | | |
| Zinkoxid | | | | | 2 | |
| Drometrizole Trisiloxane | | | | | 3 | |
| Ethylhexylmethoxycinnamat | | 6 | 5 | | | |
| Phenylbenzimidazol Sulfonsäure | | 0,5 | 2 | | 1 | |
| Homosalat | 5 | | | 7 | | |
| Butyl Methoxydibenzoylmethan | | 3 | | | | |
| Formel X (Tinsorb S) | | 2 | 3 | | | |
| Formel XVI (Uvasorb Heb) | 1,5 | 1,5 | 1,5 | 1,5 | 1,5 | 1,5 |
| Octylsalicylat | | | | 5 | | |
| Octocrylen | 1,0 | | | | 3 | |
| 3-(4-tert-Butyl-phenyl)-3-hydroxy-1-(4-methoxy-phenyl)-propan-1-on | | 1,5 | 0,5 | 2,5 | 1,0 | 5,0 |
| 1-(4-tert-Butyl-phenyl)-3-hydroxy-3-(4-methoxy-phenyl)-propan-1-on | 3,0 | | 2,5 | | | |
| Parsol^{®} SLX | 4 | | | | | 5 |
| PVP Hexadecen Copolymer | 0,5 | | 1 | | 0,8 | |
| Coenzym Q 10 | 0,2 | 0,02 | | 0,3 | | |
| Vitamin E Acetat | 0,2 | | 0,3 | | 0,8 | 0,5 |
| Na₂H₂EDTA | 0,1 | | | | | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

### Beispiel X: Hydrodisperionen (Lotionen und Sprays)

| | **A** | **B** | **C** | **D** | **E** | **F** |
|---|---|---|---|---|---|---|
| Glyceryl Stearat Citrat | | 0,40 | | | | |
| Cetyl Alkohol | | | | | 2,00 | |
| Natrium Carbomer | | | | | 0,30 | |
| Acrylates/C₁₀₋₃₀ Alkyl Acrylate Crosspolymer | 0,30 | | 0,30 | 0,40 | 0.10 | 0,10 |
| Cetsareth-20 | | | 1,00 | | | |
| Xanthan Gummi | | | | 0,15 | | 0,50 |
| Dimethicon / Vinyl Dimethicon Crosspolymer | | | | 5,00 | | 3,00 |
| Formel XV (Uvasorb K2A) | | | | | 3,50 | |
| Uvinul^{®}A Plus | 0,25 | | | 0,50 | 2,00 | 1,50 |
| Butyl Methoxydibenzoylmethan | 1,20 | | 3,50 | | | |
| Formel X (Tinsosorb S) | 2,00 | 2,00 | | 0,25 | | |
| Terephthaliden Dicampher Sulfonsäure | | | | | | 0,50 |
| Dinatrium Phenyl Dibenzimidazol Tetrasulfonat | | | | | | 1,00 |
| Phenylbenzimidazol Sulfonsäure | | | 2,00 | | | |
| Ethylhexyl Methoxycinnamat | 5,00 | | 7,00 | | 5,00 | 8,00 |
| Formel XVI (Uvasorb Heb) | | | 2,00 | 2,00 | | |
| Formel XVII (Uvinul T150) | 4,00 | 3,00 | 0,5 | | 4,00 | |
| Octocrylen | | | | 10,00 | | 2,50 |
| 3-(4-tert-Butyl-phenyl)-3-hydroxy-1-(4-methoxy-phenyl)-propan-1-on | 0,25 | 1,5 | | 2,5 | 1,0 | 5,0 |
| 1-(4-tert-Butyl-phenyl)-3-hydroxy-3-(4-methoxy-phenyl)-propan-1-on | 1,75 | | 2,0 | 2,5 | | |
| C₁₂₋₁₅ Alkyl Benzoat | 2,00 | | 2,50 | | | |
| Phenethyl Benzoat | 4,00 | | | 7,50 | | 5,00 |
| C₁₈₋₃₆ Triglycerid Fettsäure | | | 1,00 | | | |
| Butylenglycol Dicaprylat/Dicaprat | | | | | 6,00 | |
| Dicaprylyl Carbonat | | 3,00 | | | | |
| Dicaprylylether | | 2,00 | | | | |
| Cyclomethicon | | | | 1,50 | | |
| Lanolin | | | | | 0,35 | |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 050 | 0,25 | 0,75 | 1,00 |
| α-Glycosylrutin | | | | | 0,25 | |
| Trinatrium EDTA | | 1,00 | 1.00 | 0,10 | 0,20 | |
| Idopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | qs. | q.s. | q.s. |
| Wasser | ad 100 | ad 100 | ad 100 | ad 100 | ad | ad 100 |
| | | | | | 100 | |
| Neutralisationsmittel (Natriumhydroxid, Kaliumhydroxid) | qs | qs | qs | qs | qs | qs |

### Beispiel XI: wässrige und wässrig/alkoholische Formulierungen

| | **A*** | **E** | **C*** | **D** | **E** | **F*** |
|---|---|---|---|---|---|---|
| Ethanol | 50 | 5 | 2 | 40 | 15 | |
| Hydroxyethylcellulose | 0.5 | | | | | |
| Acrylates/C10-30 Alkyl Acrylate Crosspolymer | | | | 0,3 | 0,6 | |
| Cocoatnidopropylbetain | | | 0,3 | | | |
| Formel XV (Uvasorb K2A) | | | | | 2 | |
| Uvinul® APlus | 5 | | | | | |
| Butyl Methoxydibenzoylmethan | 0,5 | | | 3 | | |
| Dinatrium Phenyl Dibenzimidazol Tetrasulfonat | | 2 | 1 | | | |
| Phenylbenzimidazol Sulfonsäure | | 5 | 3 | | 2 | 4 |
| Ethylhexyl Methoxycinnamat | 10 | | | | 3 | |
| Formel XVI (Uvasorb Heb) | | 1 | | 3 | | |
| Formel XVII (Uvinul T150) | | 1 | | | 2 | |
| Octocrylen | | | | 5 | | |
| 3-(4-tert-Butyl-phenyl)-3-hydroxy-1-(4-methoxy-phenyl)-propan-1-on | 2,5 | | 1,5 | 3,0 | 3,5 | 4,0 |
| 1-(4-tert-Butyl-phenyl)-3-hydroxy-3-(4-methoxy-phenyl)-propan-1-on | 2,5 | 3,0 | | | | 1,0 |

| | | | | | | |
|---|---|---|---|---|---|---|
| ***** Vergleichsbeispiel | | | | | | |

| | **A** | **E** | **C** | **D** | **E** | **F** |
|---|---|---|---|---|---|---|
| C₁₂₋₁₅ Alkyl Benzoat | | | | 3 | | |
| C18-36 Triglycerid Fettsäure | | | | 1 | | |
| Butylenglycol Dicaprylat/Dicaprat | 2 | | | | | |
| C12-13 Alkyl Tartrat | | | | | 5 | |
| Cyclomethicon | 4 | | | 2 | | |
| Insekt Repellent^{®} 3535 | | | | 5 | | |
| Dimethicon | | | | | 3 | |
| PVP Hexadecen Copolymer | | 0,5 | | 1 | | 0.5 |
| Ethylhexyloxyglycerin | | 0,5 | | | | |
| Glycerin | 5 | 7 | 3 | 8 | | S |
| Butylenglycol | | | 5 | | 5 | |
| Metylpropandiol | | | | 4 | | |
| Vitamin E Acetat | | 0,3 | 0,2 | 0,5 | | |
| Panthenol | 0,5 | | 0,2 | | | 0,3 |
| Kreatinin | | | 0,01 | | 0,02 | |
| Creatin | | | 0,1 | | 0,2 | |
| PEG-40 Hydriertes Ricinusöl | | 0,5 | 0,3 | | | 0.5 |
| Trinatrium EDTA | 0,3 | 0,2 | 0,2 | 0,2 | 0,2 | 0,5 |
| Konservierungsmittel | q.s. | q.s. | q.s. | q.s. | q.s. | q.s. |
| Natriumhydroxid | q.s. | q.s. | q.s. | q.s. | q.s. | q.s. |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

### Beispiel XII: kosmetische Schäume

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20 Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| Formel XV (Uvasorb K2A) | 0,5 | 0,5 | 2 |
| Uvinul® A Plus | 2 | 3 | |
| 3-(4-tert-Butyl-phenyl)-3-hydroxy-1-(4-methoxy-phenyl)-propan-1-on | 0,5 | 1,0 | 1,5 |
| 1-(4-tert-Butyl-phenyl)-3-hydroxy-3-(4-methoxy-phenyl)-propan-1-on | 1,5 | 1,0 | 0,5 |
| Parsol SLX^{®} | | 3 | |
| Homosalat | | 5 | |
| Phenylbenzimidazol Sulfonsäure | | 2 | 2 |
| Benzophenon-3 | 2 | | |
| Octylsalicylat | | 5 | |
| Octocrylen | 2 | | |
| Formel X (Tinsosorb S) | | 3 | |
| 2,2'-Methylen-bis-(6-(2H-benzotriazol-2-yl)-4-(1,1,3,3-tetramethylbutyl)-phenol) | | | 8 |
| Formel XII | 5 | | 4 |
| C8-C16 Alkylpolyglycoside | 1 | | |
| Vitamin E Acetat | 0,6 | 0,5 | 0,2 |
| Kreatin/Kreatinin | | | 0,5 |
| BHT | | | 0,1 |
| Na₂H₂EDTA | 0,50 | | |
| Parfum, Konservierungsmitte | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

### Beispiel XIII: kosmetische Schäume

| **Emulsion** | **D** | **E** | **F** | **G** |
|---|---|---|---|---|
| Stearinsäure | 2 | | | |
| Palmitinsäure | | | 3 | 3 |
| Cetylalkohol | 2 | 2 | | |
| Cetylstearylalkohol | | | 2 | 2 |
| Stearylalkohol | | | | |
| PEG-100 Stearat | | 4 | | |
| PEG-40 Stearat | 2 | | | |
| PEG-20 Stearat | | | 3 | 3 |
| Sorbitanstearat | 0,8 | | | |
| Tridecyl Trimellitate | | 5 | | |
| C₁₂₋₁₅ Alkyl Benzoat | | | 3 | 3 |
| Butylenglycol Dicaprylat/Dicaprat | 8 | | | |
| Octyldodecanol | | 2 | | |
| Cocoglyceride | | | | 2 |
| Dicaprylyl Ether | | | 2 | 2 |
| Cyclomethicon | | | | |
| Dimethicon | 1 | | 2 | 2 |
| Isohexadecan | | 3 | | |
| Methylpropandiol | | 4 | | |
| Propylenglykol | | | | |
| Glycerin | 5 | | 6 | 6 |
| NeoHeliopan^{®} AP | | 2 | | |
| Phenylbenzimidazol | 1 | | | 1 |
| sulfonsäure | | | | |
| 3-(4-tert-Butyl-phenyl)-3-hydroxy-1-(4-methoxy-phenyl)-propan-1-on | | 1,5 | 3,0 | 6,0 |
| 1-(4-tert-Butyl-phenyl)-3-hydroxy-3-(4-methoxy-phenyl)-propan-1-on | 2,0 | 2,0 | 2,0 | 2,0 |
| Ethylhexylmethoxycinnama t | 5 | | 4 | 4 |
| Formel XVII (Uvinul T150) | | 2 | | 1 |
| Eusolex T-AVO^{®} | 2 | | | |
| Formel XVI (Uvasorb Heb) | 1 | | 1,0 | |
| Butyl Methoxydibenzoylmethan | 2,5 | | 2 | 2 |
| Formel X (Tinsoreb S) | 2 | 1,5 | 1,0 | |
| Vitamin E Acetat | 0,2 | | 0,3 | 0,3 |
| Na₂H₂EDTA | | | | |
| Parfum, Konservierungsmitte | | | | |
| Farbstoffe, usw. | | | | |
| Natriumhydroxid | | q-s. | q.s. | |
| Triethanolamin | q.s. | | | q.s. |
| Wasser | ad 100,0 | ad 100,0 | ad 100,0 | ad 100,0 |

## Patentansprüche

1. Verwendung zumindest einer Verbindung nach Formel I 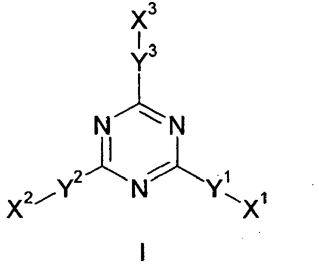
wobei Y¹, Y² und Y³ jeweils unabhängig voneinander stehen für eine Einfachbindung oder NH,
wobei X¹, X² und X³ jeweils unabhängig voneinander stehen für Alk¹ oder einen Substituenten der Formel II oder III 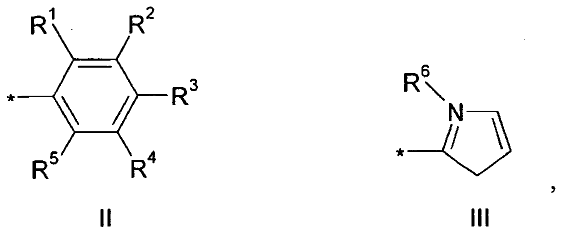
wobei R¹ bis R⁵ jeweils unabhängig voneinander stehen für H, OH, Hal, Alk¹, OAlk¹, SAlk¹, NHAlk¹, N(Alk¹)₂, COOAlk¹, COOH, C(O)H, CONHAlk¹, CONH₂, COO⁻Kt⁺, Cyc¹, OCyc¹, Arl¹, OArl¹, COOArl¹, Biphenylyl, Het¹, OHet¹, Si(Alk²)₃, OEth¹, COOEth¹ oder
für einen Substituenten der Formel IV 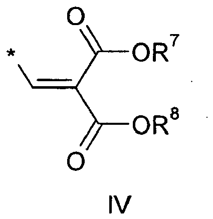
wobei Alk¹ jeweils unabhängig voneinander steht
für eine geradkettige oder verzweigte C₁- bis C₂₀-Alkylgruppe oder für eine geradkettige oder verzweigte C₂- bis C₂₀-Alkenylgruppe, die zumindest eine Doppelbindung aufweisen kann oder für eine geradkettige oder verzweigte C₂- bis C₂₀-Alkinylgruppe, die zumindest eine Dreifachbindung aufweisen kann, und/oder bei der zumindest ein oder mehrere nicht benachbarte C-Atome der Alkyl-, Alkenyl- oder Alkinylgruppe durch O oder Trimethylsilyl ersetzt sein kann/können und/oder die zumindest ein an ein primäres oder sekundäres C-Atom angebundenes OH aufweisen können,
wobei Cyc¹ jeweils unabhängig voneinander steht für eine C₃- bis C₈-Cycloalkyl-Gruppe, die zumindest eine Doppelbindung aufweisen kann, und/oder in der zumindest ein CH₂ durch O oder NH ersetzt sein kann,
wobei Arl¹ jeweils unabhängig voneinander steht für eine unsubstituierte, einfach- oder mehrfachsubstituierte C₆- bis C₂₀-Arylgruppe,
wobei Het¹ jeweils unabhängig voneinander steht für eine unsubstituierte, einfach- oder mehrfachsubstituierte C₅- bis C₂₀-Arylgruppe, bei der zumindest ein CH₂ durch O, S oder NH ersetzt ist,
wobei Alk² jeweils unabhängig voneinander steht für eine geradkettige oder verzweigte C₁- bis C₁₂-Alkylgruppe, die durch zumindest ein O unterbrochen sein kann,
wobei R⁶ steht für Alk¹,
wobei R⁷ und R⁸ jeweils unabhängig voneinander stehen
für Alk¹ oder Arl¹,
wobei Eth¹ jeweils unabhängig voneinander steht
für -(CH₂-CH₂-O)ₘ₁-Alk² mit 1 ≤ m¹ ≤ 16,
wobei Hal steht für F, Cl, Br oder I,
wobei Kt⁺ steht für Li⁺, Na⁺ oder K⁺,
und/oder deren Salz als Beschleuniger für eine photoinduzierte Reaktion von 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen zu 1-(4-tert-Butylphenyl)-3-(4-methoxyphenyl)propan-1,3-dion.

2. Verwendung nach Anspruch 1,
**dadurch gekennzeichnet, dass** in Formel I X¹ und X² jeweils unabhängig voneinander für einen Substituenten der Formel II stehen und X³ für Alk¹ oder unabhängig von X¹ und X² für einen Substituenten der Formel II steht.

3. Verwendung nach Anspruch 1 oder 2,
**dadurch gekennzeichnet, dass** die Substituenten R² und R⁵ in Formel II H bedeuten.

4. Verwendung nach einem oder mehreren der Ansprüche 1 bis 3,
**dadurch gekennzeichnet, dass** die Substituenten R¹, R³ und R⁴ jeweils unabhängig voneinander stehen für H OH, Alk¹, OAlk¹, Arl¹, Het¹, Biphenylyl, COOAlk¹, CONHAlk¹ oder einen Substituenten der Formel IV

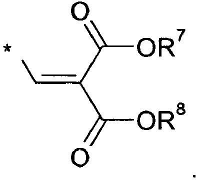

5. Verwendung nach einem oder mehreren der Ansprüche 1 bis 4, **dadurch gekennzeichnet, dass** in Formel I Y³-X³ jeweils unabhängig voneinander die Kombinationen umfasst ausgewählt aus der Gruppe Y³ steht für NH und X³ steht für Alk¹, Y³ steht für eine Einfachbindung und X³ steht für einen Substituenten der Formel II, wobei R¹, R², R⁴ und R⁵ H bedeuten und R³ OAlk¹, Arl¹ oder Biphenylyl bedeutet oder Y³ steht für NH und X³ steht für einen Substituenten der Formel II, wobei R¹ H oder Alk¹ bedeutet, R² und R⁵ H bedeuten, R⁴ H oder OH bedeutet und R³ COOAlk¹, CONHAlk¹, Het¹ oder einen Substituenten der Formel IV bedeutet, wobei R⁷ oder R⁸ jeweils unabhängig voneinander Alk¹ bedeuten.

6. Verwendung nach einem oder mehreren der Ansprüche 1 bis 5, **dadurch gekennzeichnet, dass** in Formel I Y¹-X¹ und Y²-X² jeweils unabhängig voneinander die Kombinationen umfasst ausgewählt aus der Gruppe Y¹ und Y² stehen für eine Einfachbindung und X¹ und X² stehen jeweils unabhängig voneinander für einen Substituenten der Formel II, wobei R¹ H, OAlk¹ oder OH bedeutet, R², R⁴ und R⁵ H bedeuten und R³ Arl¹, OAlk¹, Alk¹ oder Biphenylyl bedeutet oder Y¹ und Y² stehen für NH und X¹ und X² stehen jeweils unabhängig voneinander für einen Substituenten der Formel II, wobei R¹ H oder OH bedeutet, R² und R⁵ H bedeuten, R⁴ H oder Alk¹ bedeutet und R³ COOAlk¹, Het¹ oder einen Substituenten der Formel IV bedeutet, wobei R⁷ oder R⁸ jeweils unabhängig voneinander Alk¹ bedeuten.

7. Verwendung mindestens einer Verbindung der Formel I, wie in einem oder mehreren der Ansprüche 1 bis 6 beschrieben, zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen zur Herstellung kosmetischer und/oder dermatologischer und/oder pharmazeutischer Zubereitungen und/oder von Nahrungsmitteln und/oder von Nahrungsergänzungsmitteln und/oder Kunststoffen.

8. Verwendung nach einem oder mehreren der Ansprüche 1 bis 7,
**dadurch gekennzeichnet, dass**
3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen zusammen mit zumindest einer Verbindung der Formel I, wie in einem oder mehreren der Ansprüche 1 bis 6 beschrieben, in Kombination mit zumindest einem weiteren UV-Filter verwendet wird.

9. Verwendung einer Mischung aus zumindest einer Verbindung der Formel I, wie in einem oder mehreren der Ansprüche 1 bis 6 beschrieben, zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen, als photoinduziert-antioxidatives System.

10. Verwendung nach Anspruch 9,
**dadurch gekennzeichnet, dass** die Mischung als photoinduzierter Stabilisator für einen UV-Filter verwendet wird.

11. Verwendung einer Mischung aus zumindest einer Verbindung der Formel I, wie in einem oder mehreren der Ansprüche 1 bis 6 beschrieben, zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen und zumindest einem weiteren UV-Filter als UV-Filtersystem für eine Zubereitung, wobei sich die Zubereitung mit einem solchen UV-Filtersystem versehen bei Bestrahlung mit Sonnen- oder sonnenähnlichem Licht in einem vorbestimmten Zeitraum durch eine im Wesentlichen gleichbleibende oder ansteigende UV-Absorbtionsfähigkeit auszeichnet.

12. Zubereitung enthaltend zumindest einen für kosmetische, pharmazeutische, dermatologische Zubereitungen, Nahrungsmittel, Nahrungsergänzungsmittel, Haushaltsprodukte oder Kunststoffe geeigneten Träger, gegebenenfalls einen weiteren UV-Filter und eine Mischung aus zumindest einer Verbindung nach Formel I, wie in einem oder mehreren der Ansprüche 1 bis 6 beschrieben und 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen.

13. Zubereitung nach Anspruch 12,
**dadurch gekennzeichnet, dass** die Zubereitung während Bestrahlung mit Sonnen- oder sonnenähnlichem Licht innerhalb eines vorbestimmten Zeitraums maximal eine um +/- 5% abweichende UV-Absorbtionsfähigkeit aufweist.

14. Zubereitung nach Anspruch 12 oder 13,
**dadurch gekennzeichnet, dass** die Zubereitung während Bestrahlung mit Sonnen- oder sonnenähnlichem Licht innerhalb eines vorbestimmten Zeitraums eine ansteigende UV-Absorbtionsfähigkeit aufweist.

15. Verfahren zur Stabilisierung der UV-Absorbtionsfähigkeit einer Zubereitung mittels einer Mischung aus zumindest einer Verbindung nach Formel I, wie in einem oder mehreren der Ansprüche 1 bis 6 beschrieben, zusammen mit 3-(4-tert-Butyl-phenyl)-3-hydroxy-1-(4-methoxy-phenyl)-propan-1-on, 1-(4-tert-Butyl-phenyl)-3-hydroxy-3-(4-methoxy-phenyl)-propan-1-on oder einer Mischung dieser beiden Verbindungen, wobei die Mischung und die Zubereitung so aufeinander abgestimmt sind, dass bei Bestrahlung die UV-Absorbtionsfähigkeit der Zubereitung in einem vorbestimmten Zeitraum eine Abweichung von maximal +/- 5% aufweist oder ansteigt.

## Claims

1. Use of at least one compound of the formula I 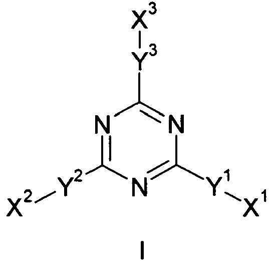
where Y¹, Y² and Y³ each stand, independently of one another, for a single bond or NH,
where X¹, X² and X³ each stand, independently of one another, for Alk¹ or a substituent of the formula II or III 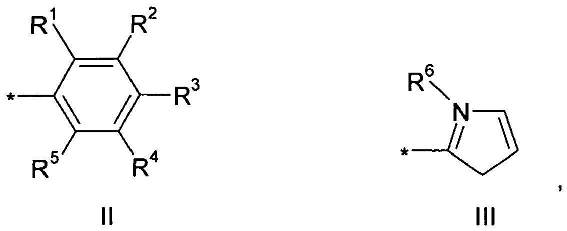
where R¹ to R⁵ each stand, independently of one another,
for H, OH, Hal, Alk¹, OAlk¹, SAlk¹, NHAlk¹, N(Alk¹)₂, COOAlk¹, COOH, C(O)H, CONHAlk¹, CONH₂, COO⁻Kt⁺, Cyc¹, OCyc¹, Arl¹, OArl¹, COOArl¹, biphenylyl, Het¹, OHet¹, Si(Alk²)₃, OEth¹, COOEth¹ or for a substituent of the formula IV 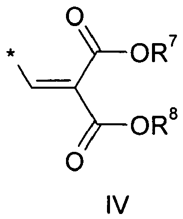
where Alk¹ in each case stands, independently of one another,
for a straight-chain or branched C₁- to C₂₀-alkyl group or for a straight-chain or branched C₂- to C₂₀-alkenyl group, which may have at least one double bond, or for a straight-chain or branched C₂- to C₂₀-alkynyl group, which may have at least one triple bond, and/or in which at least one or more non-adjacent C atoms of the alkyl, alkenyl or alkynyl group may be replaced by O or trimethylsilyl and/or which may contain at least one OH bonded to a primary or secondary C atom,
where Cyc¹ in each case stands, independently of one another, for a C₃- to C₈-cycloalkyl group, which may have at least one double bond, and/or in which at least one CH₂ may be replaced by O or NH, where Arl¹ in each case stands, independently of one another, for an unsubstituted, mono- or polysubstituted C₆- to C₂₀-aryl group, where Het¹ in each case stands, independently of one another, for an unsubstituted, mono- or polysubstituted C₅- to C₂₀-aryl group, in which at least one CH₂ has been replaced by O, S or NH, where Alk² in each case stands, independently of one another, for a straight-chain or branched C₁- to C₁₂-alkyl group, which may be interrupted by at least one O,
where R⁶ stands for Alk¹,
where R⁷ and R⁸ each stand, independently of one another,
for Alk¹ or Arl¹,
where Eth¹ in each case stands, independently of one another,
for -(CH₂-CH₂-O)ₘ₁-Alk² where 1 ≤ m1 ≤ 16,
where Hal stands for F, Cl, Br or I,
where Kt⁺ stands for Li⁺, Na⁺ or K⁺,
and/or salt thereof as accelerator for a photoinduced reaction of 3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds to give 1-(4-tert-butylphenyl)-3-(4-methoxyphenyl)propane-1,3-dione.

2. Use according to Claim 1,
**characterised in that**, in formula I, X¹ and X² each stand, independently of one another, for a substituent of the formula II and X³ stands for Alk¹ or, independently of X¹ and X², for a substituent of the formula II.

3. Use according to Claim 1 or 2,
**characterised in that** the substituents R² and R⁵ in formula II denote H.

4. Use according to one or more of Claims 1 to 3,
**characterised in that** the substituents R¹, R³ and R⁴ each stand, independently of one another, for H OH, Alk¹, OAlk¹, Arl¹, Het¹, biphenylyl, COOAlk¹, CONHAlk¹ or a substituent of the formula IV

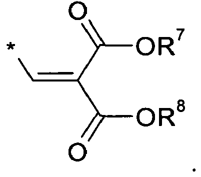

5. Use according to one or more of Claims 1 to 4, **characterised in that**, in formula I, Y³-X³ in each case, independently of one another, comprises the combinations selected from the group Y³ stands for NH and X³ stands for Alk¹, Y³ stands for a single bond and X³ stands for a substituent of the formula II, where R¹, R², R⁴ and R⁵ denote H and R³ denotes OAlk¹, Arl¹ or biphenylyl, or Y³ stands for NH and X³ stands for a substituent of the formula II, where R¹ denotes H or Alk¹, R² and R⁵ denote H, R⁴ denotes H or OH and R³ denotes COOAlk¹, CONHAlk¹, Het¹ or a substituent of the formula IV, where R⁷ or R⁸ each, independently of one another, denote Alk¹.

6. Use according to one or more of Claims 1 to 5, **characterised in that**, in formula I, Y¹-X¹ and Y²-X² each, independently of one another, comprises the combinations selected from the group Y¹ and Y² stand for a single bond and X¹ and X² each stand, independently of one another, for a substituent of the formula II, where R¹ denotes H, OAlk¹ or OH, R², R⁴ and R⁵ denote H and R³ denotes Arl¹, OAlk¹, Alk¹ or biphenylyl, or Y¹ and Y² stand for NH and X¹ and X² each stand, independently of one another, for a substituent of the formula II, where R¹ denotes H or OH, R² and R⁵ denote H, R⁴ denotes H or Alk¹ and R³ denotes COOAlk¹, Het¹ or a substituent of the formula IV, where R¹ or R⁸ each, independently of one another, denote Alk¹.

7. Use of at least one compound of the formula I, as described in one or more of Claims 1 to 6, together with 3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds for the preparation of cosmetic and/or dermatological and/or pharmaceutical preparations and/or foods and/or food supplements and/or plastics.

8. Use according to one or more of Claims 1 to 7,
**characterised in that**
3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds together with at least one compound of the formula I, as described in one or more of Claims 1 to 6, is used in combination with at least one further UV filter.

9. Use of a mixture of at least one compound of the formula I, as described in one or more of Claims 1 to 6, together with 3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds, as photoinduced-antioxidative system.

10. Use according to Claim 9,
**characterised in that** the mixture is used as photoinduced stabiliser for a UV filter.

11. Use of a mixture of at least one compound of the formula I, as described in one or more of Claims 1 to 6, together with 3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds and at least one further UV filter as UV filter system for a preparation, where the preparation provided with a UV filter system of this type is distinguished by an essentially constant or increasing UV absorption capacity in a predetermined period on irradiation with sunlight or sun-like light.

12. Preparation comprising at least one vehicle which is suitable for cosmetic, pharmaceutical, dermatological preparations, foods, food supplements, household products or plastics, optionally a further UV filter and a mixture of at least one compound of the formula I, as described in one or more of Claims 1 to 6, and 3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds.

13. Preparation according to Claim 12,
**characterised in that** the preparation has at most a UV absorption capacity differing by +/- 5% within a predetermined period during irradiation with sunlight or sun-like light.

14. Preparation according to Claim 12 or 13,
**characterised in that** the preparation has an increasing UV absorption capacity within a predetermined period during irradiation with sunlight or sun-like light.

15. Method for the stabilisation of the UV absorption capacity of a preparation by means of a mixture of at least one compound of the formula I, as described in one or more of Claims 1 to 6, together with 3-(4-tert-butylphenyl)-3-hydroxy-1-(4-methoxyphenyl)propan-1-one, 1-(4-tert-butylphenyl)-3-hydroxy-3-(4-methoxyphenyl)propan-1-one or a mixture of these two compounds, where the mixture and the preparation are matched to one another in such a way that the UV absorption capacity of the preparation has a deviation of at most +/- 5% or increases in a predetermined period on irradiation.

## Revendications

1. Utilisation d'au moins un composé de formule I 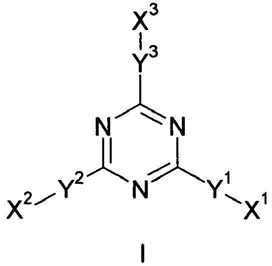
dans laquelle Y¹, Y² et Y³ représentent chacun, indépendamment les uns des autres, une liaison simple ou NH,
dans laquelle X¹, X² et X³ représentent chacun, indépendamment les uns des autres, Alk¹ ou un substituant de formule II ou III 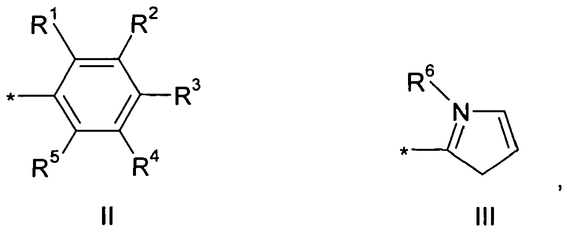
dans laquelle R¹ à R⁵ représentent chacun, indépendamment les uns des autres,
H, OH, Hal, Alk¹, OAlk¹, SAlk¹, NHAlk¹, N(Alk¹)₂, COOAlk¹, COOH, C(O)H, CONHAlk¹, CONH₂, COO⁻Kt⁺, Cyc¹, OCyc¹, Arl¹, OArl¹, COOArl¹, biphénylyle, Hét¹, OHét¹, Si(Alk²)₃, OÉth¹, COOÉth¹ ou un substituant de formule IV 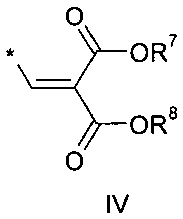
dans laquelle Alk¹ représente dans chaque cas, indépendamment les uns des autres,
un groupement C₁- à C₂₀-alkyle à chaîne linéaire ou ramifiée ou un groupement C₂- à C₂₀-alcényle à chaîne linéaire ou ramifiée, pouvant avoir au moins une double liaison, ou un groupement C₂- à C₂₀-alcynyle à chaîne linéaire ou ramifiée, pouvant avoir au moins une triple liaison, et/ou où au moins un ou plusieurs atomes de C non adjacents du groupement alkyle, alcényle ou alcynyle peuvent être remplacés par O ou triméthylsilyle et/ou pouvant contenir au moins un OH lié à un atome de C primaire ou secondaire,
où Cyc¹ représente dans chaque cas, indépendamment les uns des autres, un groupement C₃- à C₈-cycloalkyle, pouvant avoir au moins une double liaison, et/ou où au moins un CH₂ peut être remplacé par O ou NH,
où Arl¹ représente dans chaque cas, indépendamment les uns des autres, un groupement C₆- à C₂₀-aryle non substitué, mono- ou polysubstitué,
où Hét¹ représente dans chaque cas, indépendamment les uns des autres, un groupement C₅- à C₂₀-aryle non substitué, mono- ou polysubstitué, où au moins un CH₂ a été remplacé par O, S ou NH, où Alk² représente dans chaque cas, indépendamment les uns des autres, un groupement C₁- à C₁₂-alkyle à chaîne linéaire ou ramifiée, pouvant être interrompu par au moins un O,
où R⁶ représente Alk¹,
où R⁷ et R⁸ représentent chacun, indépendamment les uns des autres, Alk¹ ou Arl¹,
où Éth¹ représente dans chaque cas, indépendamment les uns des autres, -(CH₂-CH₂-O)ₘ₁-Alk² où 1 ≤ m1 ≤16,
où Hal représente F, CI, Br ou I,
où Kt⁺ représente Li⁺, Na⁺ ou K⁺,
et/ou un sel de celui-ci comme accélérateur d'une réaction photo-induite de 3-(4-tertio-butylphényl)-3-hydroxy-1-(4-méthoxyphényl)-propan-1-one, de 1-(4-tertio-butylphényl)-3-hydroxy-3-(4-méthoxy-phényl)propan-1-one ou d'un mélange de ces deux composés pour donner la 1-(4-tertio-butylphényl)-3-(4-méthoxyphényl)propane-1,3-dione.

2. Utilisation selon la revendication 1,
**caractérisée en ce que**, dans la formule I, X¹ et X² représentent chacun, indépendamment les uns des autres, un substituant de formule II et X³ représente Alk¹ ou, indépendamment de X¹ et X², un substituant de formule II.

3. Utilisation selon la revendication 1 ou 2,
**caractérisée en ce que** les substituants R² et R⁵ dans la formule II désignent H.

4. Utilisation selon l'une ou plusieurs parmi les revendications 1 à 3,
**caractérisée en ce que** les substituants R¹, R³ et R⁴ représentent chacun, indépendamment les uns des autres, H, OH, Alk¹, OAlk¹, Arl¹, Hét¹, biphénylyle, COOAlk¹, CONHAlk¹ ou un substituant de formule IV

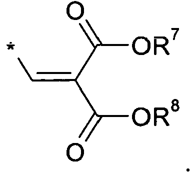

5. Utilisation selon l'une ou plusieurs parmi les revendications 1 à 4, **caractérisée en ce que**, dans la formule I, Y³-X³ dans chaque cas, indépendamment les uns des autres, comprennent les combinaisons choisies dans le groupe constitué par Y³ représente NH et X³ représente Alk¹, Y³ représente une liaison simple et X³ représente un substituant de formule II, où R¹, R², R⁴ et R⁵ désignent H et R³ désigne OAlk¹, Arl¹ ou biphénylyle, ou Y³ représente NH et X³ représente un substituant de formule II, où R¹ désigne H ou Alk¹, R² et R⁵ désignent H, R⁴ désigne H ou OH et R³ désigne COOAlk¹, CONHAlk¹, Hét¹ ou un substituant de formule IV, où R⁷ ou R⁸ désignent chacun, indépendamment l'un de l'autre, Alk¹.

6. Utilisation selon l'une ou plusieurs parmi les revendications 1 à 5, **caractérisée en ce que**, dans la formule I, Y¹-X¹ et Y²-X² comprennent chacun, indépendamment les uns des autres, les combinaisons choisies dans le groupe constitué par Y¹ et Y² représentent une liaison simple et X¹ et X² représentent chacun, indépendamment l'un de l'autre, un substituant de formule II, où R¹ désigne H, OAlk¹ ou OH, R², R⁴ et R⁵ désignent H et R³ désigne Arl¹, OAlk¹, Alk¹ ou biphénylyle, ou Y¹ et Y² représentent NH et X¹ et X² représentent chacun, indépendamment l'un de l'autre, un substituant de formule II, où R¹ désigne H ou OH, R² et R⁵ désignent H, R⁴ désigne H ou Alk¹ et R³ désigne COOAlk¹, Hét¹ ou un substituant de formule IV, où R⁷ ou R⁸ désignent chacun, indépendamment l'un de l'autre, Alk¹.

7. Utilisation d'au moins un composé de formule I, tel que décrit selon l'une ou plusieurs parmi les revendications 1 à 6, conjointement avec de la 3-(4-tertio-butylphényl)-3-hydroxy-1-(4-méthoxyphényl)propan-1-one, de la 1-(4-tertio-butylphényl)-3-hydroxy-3-(4-méthoxyphényl)-propan-1-one ou un mélange de ces deux composés, pour la préparation de préparations cosmétiques et/ou dermatologiques et/ou pharmaceutiques et/ou d'aliments et/ou de compléments alimentaires et/ou de matières plastiques.

8. Utilisation selon l'une ou plusieurs parmi les revendications 1 à 7,
**caractérisée en ce que**
la 3-(4-tertio-butylphényl)-3-hydroxy-1-(4-méthoxyphényl)propan-1-one, la 1-(4-tertio-butylphényl)-3-hydroxy-3-(4-méthoxyphényl)-propan-1-one ou un mélange de ces deux composés, conjointement avec au moins un composé de formule I, tel que décrit selon l'une ou plusieurs parmi les revendications 1 à 6, est utilisé en combinaison avec au moins un autre filtre anti-UV.

9. Utilisation d'un mélange d'au moins un composé de formule I, tel que décrit selon l'une ou plusieurs parmi les revendications 1 à 6, conjointement avec de la 3-(4-tertio-butylphényl)-3-hydroxy-1-(4-méthoxyphényl)propan-1-one, de la 1-(4-tertio-butylphényl)-3-hydroxy-3-(4-méthoxyphényl)propan-1-one ou un mélange de ces deux composés, comme système antioxydant photo-induit.

10. Utilisation selon la revendication 9,
**caractérisée en ce que** le mélange est utilisé comme stabilisant photo-induit pour un filtre anti-UV.

11. Utilisation d'un mélange d'au moins un composé de formule I, tel que décrit selon l'une ou plusieurs parmi les revendications 1 à 6, conjointement avec de la 3-(4-tertio-butylphényl)-3-hydroxy-1-(4-méthoxyphényl)propan-1-one, de la 1-(4-tertio-butylphényl)-3-hydroxy-3-(4-méthoxyphényl)propan-1-one ou un mélange de ces deux composés, et d'au moins un autre filtre anti-UV comme système de filtre anti-UV pour une préparation, où la préparation pourvue d'un système de filtre anti-UV de ce type est **caractérisée par** une capacité d'absorption d'UV sensiblement constante ou croissante dans une période prédéterminée lors d'une irradiation par de la lumière du soleil ou de la lumière semblable à celle du soleil.

12. Préparation comprenant au moins un véhicule qui est convenable pour les préparations cosmétiques, pharmaceutiques, dermatologiques, les aliments, les compléments alimentaires, les produits ménagers ou les matières plastiques, éventuellement un autre filtre anti-UV et un mélange d'au moins un composé de formule I, tel que décrit selon l'une ou plusieurs parmi les revendications 1 à 6, et de 3-(4-tertio-butylphényl)-3-hydroxy-1-(4-méthoxyphényl)propan-1-one, de 1-(4-tertio-butylphényl)-3-hydroxy-3-(4-méthoxyphényl)propan-1-one ou d'un mélange de ces deux composés.

13. Préparation selon la revendication 12,
**caractérisée en ce que** la préparation présente au plus une capacité d'absorption d'UV qui diffère de +/- 5% au sein d'une période prédéterminée lors d'une irradiation par de la lumière du soleil ou de la lumière semblable à celle du soleil.

14. Préparation selon la revendication 12 ou 13,
**caractérisée en ce que** la préparation possède une capacité d'absorption d'UV accrue au sein d'une période prédéterminée lors d'une irradiation par de la lumière du soleil ou de la lumière semblable à celle du soleil.

15. Méthode de stabilisation de la capacité d'absorption d'UV d'une préparation à l'aide d'un mélange d'au moins un composé de formule I, tel que décrit selon l'une ou plusieurs parmi les revendications 1 à 6, conjointement avec de la 3-(4-tertio-butylphényl)-3-hydroxy-1-(4-méthoxyphényl)propan-1-one, de la 1-(4-tertio-butylphényl)-3-hydroxy-3-(4-méthoxyphényl)propan-1-one ou un mélange de ces deux composés, où le mélange et la préparation sont ajustés l'un à l'autre de telle sorte que la capacité d'absorption d'UV de la préparation présente un écart d'au plus +/- 5% ou augmente dans une période prédéterminée lors d'une irradiation.
